# EUROPEAN PATENT APPLICATION

(11) **EP 2 708 537 A1**
(43) Date of publication of application: **19.03.2014**
(21) Application number: 12781685.8
(22) Date of filing: 08.05.2012
(51) Int. Cl.: C07D 401/14, A61K 31/4192, A61K 31/422, A61K 31/4245, A61K 31/4439, A61K 31/506, A61P 1/00, A61P 5/00, A61P 9/12, A61P 25/08, A61P 25/16, A61P 25/20, A61P 25/22, A61P 25/24

(54) **HETEROAROMATIC RING DERIVATIVE**

(30) Priority: 10.05.2011 JP 2011105188
(71) Applicant: Taisho Pharmaceutical Co., Ltd., Tokyo 170-8633 (JP)
(72) Inventor: SUZUKI Ryo, Tokyo 170-8633 (JP); FUTAMURA Aya, Tokyo 170-8633 (JP); ABE Masahito, Tokyo 170-8633 (JP); KASHIWA Shuhei, Tokyo 170-8633 (JP); HATTORI Nobutaka, Tokyo 170-8633 (JP); NOZAWA Dai, Tokyo 170-8633 (JP); OHTA Hiroshi, Tokyo 170-8633 (JP); ARAKI Yuko, Tokyo 170-8633 (JP)
(74) Representative: HOFFMANN EITLE
(86) International application number: PCT/JP2012/061745
(87) International publication number: WO 2012/153729

(57) **Abstract**

The compound represented by formula (IA) 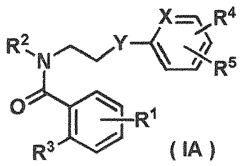 or a pharmaceutically acceptable salt thereof is based on orexin (OX) receptor antagonist activity, is useful in the treatment and prevention of illnesses including sleep disorder, depression, anxiety disorder, panic disorder, schizophrenia, drug dependency, Alzheimer's disease, Parkinson's disease, Huntington's chorea, eating disorders, pain, gastrointestinal disease, epilepsy, inflammation, immunological disease, endocrinological related disease, and hypertension.

## Description

### TECHNICAL FIELD

The present invention relates to a compound having orexin (OX) receptor antagonist activity, a pharmaceutically acceptable salt thereof, and a therapeutic or prophylactic agent containing the compound or the salt as an active ingredient for a disease such as sleep disorder, depression, anxiety disorder, panic disorder, schizophrenia, drug dependence, Alzheimer's disease, Parkinson's disease, Huntington's disease, eating disorders, pain, digestive system disease, epilepsy, inflammation, immune related disease, endocrine related disease, or hypertension.

### BACKGROUND ART

Orexin is a neuropeptide produced by splicing of prepro-orexin that is specifically expressed in the lateral hypothalamic area. Until now, OX-A composed of 33 amino acids and OX-B composed of 28 amino acids have been identified. These peptides are highly involved in regulation of the sleep-wake pattern and in regulation of eating behavior.

Both OX-A and OX-B act on an OX receptor. As the OX receptor, two subtypes, OX1 and OX2 receptors, have been cloned until now. Both are known to be seventransmembrane G protein-coupled receptors mainly expressed in the brain. The OX1 receptor is specifically coupled to a G protein subclass Gq, whereas the OX2 receptor is coupled to Gq and Gi/o (see Non Patent Literatures 1 and 2).

The subtypes of the OX receptor differ from each other in tissue distribution. The OX1 receptor is highly expressed in the locus coeruleus, which is the nucleus of origin for noradrenergic neurons; on the other hand, the OX2 receptor is highly expressed in the tuberomammillary nucleus, which is the nucleus of origin for histamine neurons (see Non Patent Literatures 3 to 5). In the raphe nucleus, which is the nucleus of origin for serotonin neurons, and the ventral tegmental area, which is the nucleus of origin for dopamine neurons, both the OX1 receptor and the OX2 receptor are expressed (see Non Patent Literature 3). The orexin neurons project to the brain stem and the monoamine nervous system of the hypothalamus to provide excitatory influence on these nerves. In addition, the OX2 receptor is expressed in the acetylcholine neurons of the brain stem which is involved in regulation of REM sleep and affects the activity of these nuclei (see Non Patent Literatures 3 and 4).

Recently, the relation of the OX1 and OX2 receptors with sleep-wake regulation has attracted attention, and usefulness of compounds having OX receptor antagonist activity has been studied. Administration of OX-A into the cerebral ventricle of a rat shows, for example, enhanced spontaneous motor activity (see Non Patent Literatures 6 and 7), enhanced stereotypical behavior (see Non Patent Literature 7), and prolonged wake time (see Non Patent Literature 6). The activity of shortening the REM sleep time by OX-A administration is completely antagonized by pretreatment with an OX receptor antagonist (see Non Patent Literature 8). Furthermore, it has been reported that administration of a substance that can be orally administered and antagonizes OX1 and OX2 receptors at similar degrees decreases the motor activity, shortens the sleep latency, and increases the quantity of non-REM and REM sleep (see Non Patent Literatures 9 and 10).

As OX receptor antagonist compounds, for example, those having various structures described in Non Patent Literature 11, a review, are known.

### CITATION LIST

### NON PATENT LITERATURE

[Non Patent Literature 1] Zhu Y et al., J. Pharmacol. Sci., 92, 259-266, 2003
[Non Patent Literature 2] Zeitzer JM et al., Trends Pharmacol. Sci., 27, 368-374, 2006
[Non Patent Literature 3] Marcus JN et al., J. Comp. Neurol, 435, 6-25, 2001
[Non Patent Literature 4] Trivedi JP et al., FEBS Lett, 438, 71-75, 1998
[Non Patent Literature 5] Yamanaka A et al., Biochem. Biophys. Res. Commun., 290, 1237-1245, 2002
[Non Patent Literature 6] Hagan JJ et al., Proc. Natl. Acad. Sci. USA, 96, 10911-10916, 1999
[Non Patent Literature 7] Nakamura T et al., Brain Res., 873, 181-187, 2000
[Non Patent Literature 8] Smith MI et al., Neurosci. Lett., 341, 256-258, 2003
[Non Patent Literature 9] Brisbare-Roch C et al., Nat. Med., 13, 150-155, 2007
[Non Patent Literature 10] Cox CD et al., J. Med. Chem., 53, 5320-5332, 2010
[Non Patent Literature 11] John G et al., ChemMedChem., 5, 1197-1214, 2010

### SUMMARY OF INVENTION

### TECHNICAL PROBLEM

It is an object of the present invention to find a novel compound having OX receptor antagonist activity and to provide a therapeutic or prophylactic agent for a disease such as sleep disorder, depression, anxiety disorder, panic disorder, schizophrenia, drug dependence, Alzheimer's disease, Parkinson's disease, Huntington's disease, eating disorders, pain, digestive system disease, epilepsy, inflammation, immune related disease, endocrine related disease, or hypertension. More specifically, the object of the present invention is to provide a novel compound having excellent OX receptor antagonist activity and showing excellent pharmacokinetic properties and safety.

### SOLUTION TO PROBLEM

The present inventors have diligently investigated compounds having a novel skeleton showing antagonist activity against orexin receptors and, as a result, have found that some heteroaromatic ring derivatives represented by the following formulas have excellent OX receptor antagonist activity, and have completed the present invention.

That is, the present invention provides:
(1) A heteroaromatic ring derivative represented by formula (IA): 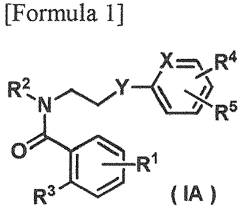 wherein,
   X represents a nitrogen atom or formula CH;
   Y represents any of the formulas in the following group (II):

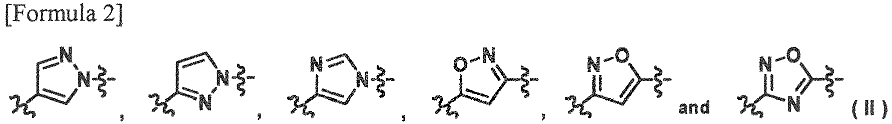

   R¹ represents a hydrogen atom, a halogen atom, or a C₁₋₆ alkyl group;
   R² represents a hydrogen atom, a C₁₋₆ alkyl group (where, the C₁₋₆ alkyl group is optionally substituted with one to three substituents selected from Substituent Group 1), a C₃₋₆ cycloalkyl group, or a 4- to 6-membered cyclic ether group, wherein
   the Substituent Group 1 is a group consisting of a halogen atom, a C₃₋₆ cycloalkyl group, and a C₁₋₆ alkoxy group;
   R³ represents a triazolyl group, a pyridyl group, or a pyrimidinyl group, wherein
   the triazolyl group, the pyridyl group, and the pyrimidinyl group are each optionally substituted with one to three halogen atoms; and
   R⁴ and R⁵ may be the same or different and each represent a hydrogen atom, a halogen atom, or a C₁₋₆ alkyl group (where, the C₁₋₆ alkyl group is optionally substituted with one to three halogen atoms), or a pharmaceutically acceptable salt thereof;
(2) The heteroaromatic ring derivative or a pharmaceutically acceptable salt thereof according to (1), wherein
   R⁴ represents a halogen atom; and
   R⁵ represents a hydrogen atom or a halogen atom;
(3) A heteroaromatic ring derivative represented by formula (I): 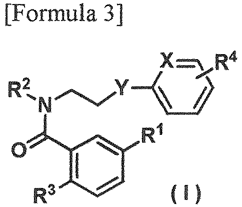 wherein,
   X represents a nitrogen atom or formula CH;
   Y represents any of the formulas in the following group (II): 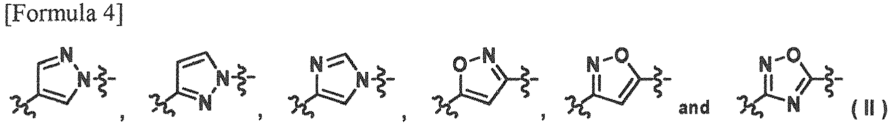
   R¹ represents a hydrogen atom, a halogen atom, or a C₁₋₆ alkyl group;
   R² represents a hydrogen atom, a C₁₋₆ alkyl group (where, the C₁₋₆ alkyl group is optionally substituted with one to three substituents selected from Substituent Group 1), a C₃₋₆ cycloalkyl group, or a 4- to 6-membered cyclic ether group, wherein
   the Substituent Group 1 is a group consisting of a halogen atom, a C₃₋₆ cycloalkyl group, and a C₁₋₆ alkoxy group;
   R³ represents a triazolyl group, a pyridyl group, or a pyrimidinyl group, wherein
   the triazolyl group, the pyridyl group, and the pyrimidinyl group are each optionally substituted with one to three halogen atoms; and
   R⁴ represents a hydrogen atom, a halogen atom, or a C₁₋₆ alkyl group, or a pharmaceutically acceptable salt thereof;
(4) The heteroaromatic ring derivative or a pharmaceutically acceptable salt thereof according to any one of (1) to (3), wherein
   X represents a nitrogen atom;
   R² represents a C₁₋₆ alkyl group;
   R³ represents a triazolyl group or a pyrimidinyl group; and
   R⁴ represents a halogen atom;
(5) The heteroaromatic ring derivative or a pharmaceutically acceptable salt thereof according to any one of (1) to (4), wherein
   R² represents a methyl group or an ethyl group;
(6) A pharmaceutical composition comprising a heteroaromatic ring derivative or a pharmaceutically acceptable salt thereof according to any one of (1) to (5) as an active ingredient; and
(7) A therapeutic or prophylactic agent for a disease such as sleep disorder, depression, anxiety disorder, panic disorder, schizophrenia, drug dependence, Alzheimer's disease, Parkinson's disease, Huntington's disease, eating disorder, pain, digestive system disease, epilepsy, inflammation, immune related disease, endocrine related disease, or hypertension, wherein the agent comprises a heteroaromatic ring derivative or a pharmaceutically acceptable salt thereof according to any one of (1) to (5) as an active ingredient.

### ADVANTAGEOUS EFFECTS OF INVENTION

It has been revealed that the heteroaromatic ring derivative of the present invention has an affinity to an OX receptor and shows antagonist activity against stimulation by a physiological ligand to the receptor.

### DESCRIPTION OF EMBODIMENTS

The terms used in the specification have the following meanings.

The term "halogen atom" is a fluorine atom, a chlorine atom, a bromine atom, or an iodine atom.

The term "C₁₋₆ alkyl group" refers to a linear or branched alkyl group having 1 to 6 carbon atoms, and examples thereof include methyl, ethyl, n-propyl, isopropyl, n-butyl, isobutyl, sec-butyl, tert-butyl, n-pentyl, isopentyl, neopentyl, tert-pentyl, 1-ethylpropyl, n-hexyl, isohexyl, and neohexyl groups.

The term "C₃₋₆ cycloalkyl group" is a cyclopropyl, cyclobutyl, cyclopentyl, or cyclohexyl group.

The term "4- to 6-membered cyclic ether group" is an oxetanyl, tetrahydrofuranyl, or tetrahydropyranyl group.

The term "C₁₋₆ alkoxy group" refers to a linear or branched alkoxy group having 1 to 6 carbon atoms, and examples thereof include methoxy, ethoxy, n-propoxy, isopropoxy, n-butoxy, isobutoxy, sec-butoxy, tert-butoxy, n-pentyloxy, isopentyloxy, neopentyloxy, tert-pentyloxy, 1-ethylpropoxy, and n-hexyloxy groups.

Throughout the specification, the term "pharmaceutically acceptable salt" refers to a pharmacologically acceptable acid addition salt, and examples of usable acids include salts with inorganic acids such as sulfuric acid, hydrochloric acid, hydrobromic acid, phosphoric acid, and nitric acid; and salts with organic acids such as acetic acid, benzoic acid, oxalic acid, lactic acid, malic acid, tartaric acid, fumaric acid, maleic acid, citric acid, malonic acid, mandelic acid, gluconic acid, galactaric acid, glucoheptonic acid, glycolic acid, glutamic acid, methanesulfonic acid, ethanesulfonic acid, benzenesulfonic acid, p-toluenesulfonic acid, camphorsulfonic acid, and naphthalene-2-sulfonic acid. The free form can be converted to a salt by a conventional method.

Preferred embodiments of the inventive compound will now be described.

In the compound, X is preferably a nitrogen atom.

In the compound, Y is preferably any of the formulas in the following group (IIa):

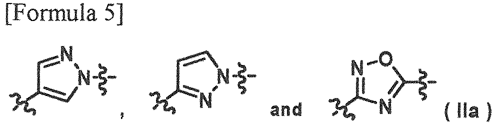

In the compound, R¹ is preferably a hydrogen atom, a halogen atom other than iodine, or a C₁₋₆ alkyl group and is more preferably a hydrogen atom, a fluorine atom, a chlorine atom, or a methyl group.

In the compound, R² is preferably a C₁₋₆ alkyl group and more preferably a methyl group or an ethyl group.

In the compound, R³ is preferably a triazolyl group or a pyrimidinyl group and is more preferably a 1,2,3-triazol-2-yl group or a pyrimidin-2-yl group.

In the compound, R⁴ is preferably a halogen atom, more preferably a halogen atom other than iodine, more preferably a fluorine atom or a chlorine atom, and most preferably a fluorine atom.

In the compound, R⁵ is preferably a hydrogen atom or a halogen atom and more preferably a hydrogen atom or a fluorine atom.

Preferred examples of the inventive compound include:
N-ethyl-N-{2-[1-(5-fluoropyridin-2-yl)-1H-pyrazol-4-yl]ethyl}-5-methyl-2-(2H-1,2,3-triazol-2-yl)benzamide,
N-{2-[1-(5-fluoropyridin-2-yl)-1H-pyrazol-4-yl]ethyl}-N,5-dimethyl-2-(2H-1,2,3-triazol-2-yl)benzamide,
N-ethyl-N-{2-[1-(5-fluoropyridin-2-yl)-1H-pyrazol-4-yl]ethyl}-2-(2H-1,2,3-triazol-2-yl)benzamide,
N-ethyl-N-{2-[1-(5-fluoropyridin-2-yl)-1H-pyrazol-3-yl]ethyl}-2-(2H-1,2,3-triazol-2-yl)benzamide,
N-{2-[1-(5-fluoropyridin-2-yl)-1H-pyrazol-3-yl]ethyl}-N,5-dimethyl-2-(2H-1,2,3-triazol-2-yl)benzamide,
N-ethyl-N-{2-[1-(5-fluoropyridin-2-yl)-1H-pyrazol-3-yl]ethyl}-5-methyl-2-(pyrimidin-2-yl)benzamide,
N-ethyl-N-{2-(1-(5-fluoropyridin-2-yl)-1H-pyrazol-3-yl]ethyl}-5-fluoro-2-(2H-1,2,3-triazol-2-yl)benzamide,
N-ethyl-N-{2-[1-(5-fluoropyridin-2-yl)-1H-pyrazol-3-yl]ethyl}-5-methyl-2-(2H-1,2,3-triazol-2-yl)benzamide,
N-ethyl-N-{2-[3-(5-fluoropyridin-2-yl)-1,2-oxazol-5-yl]ethyl}-5-methyl-2-(2H-1,2,3-triazol-2-yl)benzamide,
N-ethyl-N-{2-[3-(5-fluoropyridin-2-yl)-1,2,4-oxadiazol-5-yl]ethyl}-5-methyl-2-(2H-1,2,3-triazol-2-yl)benzamide,
5-chloro-N-{2-[1-(5-fluoropyridin-2-yl)-1H-pyrazol-3-yl]ethyl}-N-methyl-2-(2H-1,2,3-triazol-2-yl)benzamide,
N-ethyl-4-fluoro-N-{2-[1-(5-fluoropyridin-2-yl)-1H-pyrazol-3-yl]ethyl}-2-(2H-1,2,3-triazol-2-yl)benzamide,
N-ethyl-2-fluoro-N-{2-[1-(5-fluoropyridin-2-yl)-1H-pyrazol-3-yl]ethyl}-2-(2H-1,2,3-triazol-2-yl)benzamide,
N-ethyl-N-{2-[1-(5-fluoropyridin-2-yl)-1H-pyrazol-3-yl]ethyl}-2-(pyrimidin-2-yl)benzamide,
N-ethyl-5-fluoro-N-{2-[1-(5-fluoropyridin-2-yl)-1H-pyrazol-3-yl]ethyl}-2-(pyrimidin-2-yl)benzamide,
N-ethyl-2-fluoro-N-{2-[1-(5-fluoropyridin-2-yl)-1H-pyrazol-3-yl]ethyl}-6-(pyrimidin-2-yl)benzamide,
N-ethyl-N-{2-[1-(4-fluorophenyl)-1H-pyrazol-3-yl]ethyl}-2-(2H-1,2,3-triazol-2-yl)benzamide, N-ethyl-N-{2-[1-(6-methylpyridin-2-yl)-1H-pyrazol-3-yl]ethyl}-2-(2H-1,2,3-triazol-2-yl)benzamide,
N-ethyl-2-(2H-1,2,3-triazol-2-yl)-N-(2-{1-(6-(trifluoromethyl)pyridin-2-yl]-1H-pyrazol-3-yl}ethyl)benzamide,
N-{2-[1-(3,4-difluorophenyl)-1H-pyrazol-3-yl]ethyl}-N-ethyl-2-(2H-1,2,3-triazol-2-yl)benzamide,
N-ethyl-5-fluoro-N-{2-[1-(4-fluorophenyl)-1H-pyrazol-3-yl]ethyl}-2-(pyrimidin-2-yl)benzamide,
N-ethyl-5-fluoro-N-{2-[1-(6-methylpyridin-2-yl)-1H-pyrazol-3-yl]ethyl}-2-(pyrimidin-2-yl)benzamide,
N-{2-[1-(3,4-difluorophenyl)-1H-pyrazol-3-yl]ethyl}-N-ethyl-5-fluoro-2-(pyrimidin-2-yl)benzamide,
N-ethyl-N-{2-[1-(4-fluorophenyl)-1H-pyrazol-3-yl]ethyl}-2-(pyrimidin-2-yl)benzamide,
N-{2-[5-(4-fluorophenyl)-1,2,4-oxadiazol-3-yl]ethyl}-N,5-dimethyl-2-(2H-1,2,3-triazol-2-yl)benzamide,
N-{2-[5-(3,4-difluorophenyl)-1,2,4-oxadiazol-3-yl]ethyl}-N,5-dimethyl-2-(2H-1,2,3-triazol-3-yl)benzamide,
N-ethyl-N-{2-[5-(3,4-difluorophenyl)-1,2,4-oxadiazol-3-yl]ethyl}-5-methyl-2-(2H-1,2,3-triazol-2-yl)benzamide,
N-ethyl-N-{2-[5-(4-fluorophenyl)-1,2,4-oxadiazol-3-yl]ethyl}-5-methyl-2-(2H-1,2,3-triazol-2-yl)benzamide,
N-ethyl-N-{2-[5-(4-fluorophenyl)-1,2,4-oxadiazol-3-yl]ethyl}-5-methyl-2-(pyrimidin-2-yl)benzamide,
N-ethyl-5-fluoro-N-{2-[5-(4-fluorophenyl)-1,2,4-oxadiazol-3-yl]ethyl}-2-(pyrimidin-2-yl)benzamide,
N-ethyl-N-{2-[5-(4-fluorophenyl)-1,2,4-oxadiazol-3-yl]ethyl}-2-(2H-1,2,3-triazol-2-yl)benzamide, and,
N-ethyl-N-{2-[5-(3,4-difluorophenyl)-1,2,4-oxadiazol-3-yl]ethyl}-2-(2H-1,2,3-triazol-2-yl)benzamide.

If the inventive compound forms a hydrate or a solvate, such hydrate and solvate are encompassed within the scope of the present invention. Similarly, pharmaceutically acceptable salts of the hydrate or the solvate of the inventive compound are also encompassed within the scope of the present invention.

For example, all of enantiomers, diastereomers, equilibrium compounds, mixtures thereof at arbitrary ratios, and racemic forms of the compound of the present invention are encompassed within the scope of the present invention.

The compound according to the present invention includes a compound in which at least one hydrogen atom, carbon atom, nitrogen atom, oxygen atom, and fluorine atoms is replaced with a radioactive isotope or a stable isotope. These labeled compounds are useful for, for example, research of metabolism or pharmacokinetics or biological analysis as ligands of receptors.

The compounds according to the present invention can be administered orally or parenterally. The administration dosage form is, for example, a tablet, a capsule, granules, a powder, a fine powder, a troche, ointment, cream, a patch, an emulsion, a suspension, a suppository, or an injection, and these dosage forms can be produced by common formulation techniques (for example, methods described in the Japanese Pharmacopoeia Fifteenth Edition). These administration dosage forms can be appropriately selected depending on the symptoms, age, and weight of a patient and the purpose of the treatment.

These preparations can be produced by mixing a composition containing the compound of the present invention with a pharmacologically acceptable carrier, i.e., an excipient (e.g., crystalline cellulose, starch, lactose, or mannitol), a binder (e.g., hydroxypropyl cellulose or polyvinylpyrrolidone), a lubricant (e.g., magnesium stearate or talc), a disintegrator (e.g., carboxymethyl cellulose calcium), and other pharmacologically acceptable additives.

The compound of the present invention can be orally or parenterally administered to an adult patient at a dose of 0.001 to 500 mg one to several times per day. This dose can be appropriately increased or decreased depending on the type of the disease to be treated, the age, weight, symptoms, etc. of the patient.

Typical processes of producing the compounds (I) and (IA) of the present invention will be described in the following schemes A to L. The following schemes are merely exemplary processes of producing the inventive compounds, and the present invention is not limited thereto. In the following exemplary production processes, compounds may form salts that do not hinder the reactions.

### Scheme A

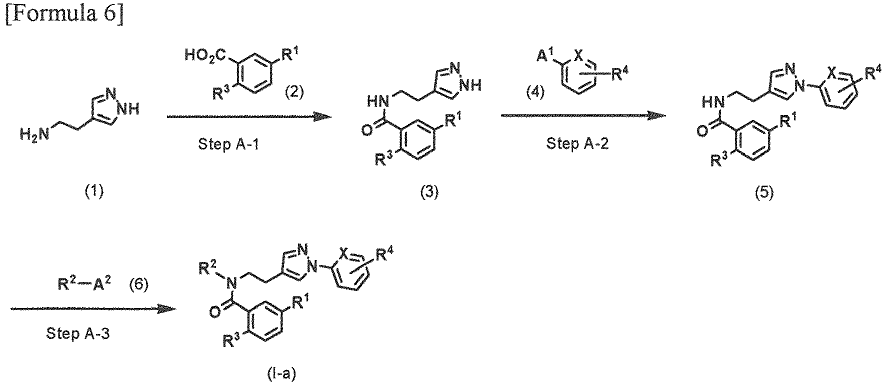

wherein, A¹ and A² each represent a halogen atom, a methanesulfonyloxy group, a p-toluenesulfonyloxy group, or a trifluoromethanesulfonyloxy group, and other variables are as defined above.
Step A-1: Compound (3) can be prepared by a condensation reaction of Compound (1) and Compound (2). The reaction in Step A-1 can be carried out by a general amidation process of a carboxylic acid. For example, the reaction is performed by a method comprising converting a carboxylic acid to a carboxylic acid halide such as carboxylic acid chloride or carboxylic acid bromide and then reacting the carboxylic acid halide with an amine or a method comprising reacting a carboxylic acid with an amine in the presence of a dehydration condensation agent. All of these reactions can be carried out in a solvent in the presence or absence of a base. Examples of the halogenating agent used in the reaction include thionyl chloride, oxalyl chloride, phosphorus oxychloride, and phosphorus oxybromide. Examples of the dehydration condensation agent used in the reaction include 1-ethyl-3-(3-dimethylaminopropyl)carbodiimide hydrochloride, propane phosphonic acid anhydride, dicyclohexylcarbodiimide, diphenylphosphonyl azide, and carbonyldiimidazole. An activator such as 1-hydroxybenzotriazole or hydroxysuccinimide can be used as needed. Examples of the solvent used in the reaction include ether solvents such as tetrahydrofuran and 1,4-dioxane; aprotic polar solvents such as N,N-dimethylformamide and acetonitrile; halogen solvents such as dichloromethane and chloroform; aromatic hydrocarbon solvents such as toluene; ethyl acetate; and mixed solvents thereof. Examples of the base used in the reaction include organic amines such as pyridine, triethylamine, and diisopropylethylamine; and inorganic bases such as potassium carbonate, sodium carbonate, and sodium bicarbonate. The reaction can be usually carried out at 0°C to 150°C, preferably at 25°C to 80°C.
Step A-2: Compound (5) can be prepared by a coupling reaction of Compound (3) and Compound (4). The reaction in Step A-2 can be carried out by a general method of introducing an aromatic ring substituent on the nitrogen atom of an azole compound using a catalyst and a ligand in the presence of a base. For example, the reaction is performed by the method described in Synlett, 2003, 15, 2428-2439 or a method that follows the method. Examples of the catalyst used in the reaction include copper catalysts such as copper (0), copper (I) iodide, copper (I) chloride, and copper (I) oxide. Examples of the ligand used in the reaction include N,N'-dimethylethylenediamine, N,N'-dimethylcyclehexane-1,2-diamine, 2-aminopyridine, 1,10-phenanthroline, and 2-hydroxybenzaldehyde oxime. Examples of the base used in the reaction include potassium carbonate, potassium phosphate, potassium hydroxide, tert-butoxy potassium, cesium carbonate, sodium carbonate, sodium bicarbonate, sodium acetate, sodium methoxide, and tetrabutylammonium hydroxide. Examples of the solvent used in the reaction include alcohol solvents such as methanol and ethanol; ether solvents such as tetrahydrofuran and 1,4-dioxane; aprotic polar solvents such as N,N-dimethylformamide, dimethyl sulfoxide, and acetonitrile; halogen solvents such as dichloromethane and chloroform; aromatic hydrocarbon solvents such as toluene; water; and mixed solvents thereof. The reaction can be usually carried out at 0°C to 150°C, preferably at 25°C to 100°C.
Step A-3: Inventive compound (I-a) can be prepared by alkylation of Compound (5). The reaction in Step A-3 can be carried out by a general method of alkylation of an amide. Examples of the base used in the reaction include inorganic bases such as sodium hydride, sodium hydroxide, sodium carbonate, potassium carbonate, and cesium carbonate; metal lower alkoxides such as sodium ethoxide and tert-butoxy potassium; and organic bases such as triethylamine and diisopropylethylamine. Examples of the solvent used in the reaction include alcohol solvents such as methanol and ethanol; ether solvents such as tetrahydrofuran and 1,4-dioxane; aprotic polar solvents such as N,N-dimethylformamide, dimethyl sulfoxide, and acetonitrile; halogen solvents such as dichloromethane and chloroform; aromatic hydrocarbon solvents such as toluene; water; and mixed solvents thereof. The reaction can be usually carried out at 0°C to 150°C, preferably at 20°C to 100°C.

### Scheme B

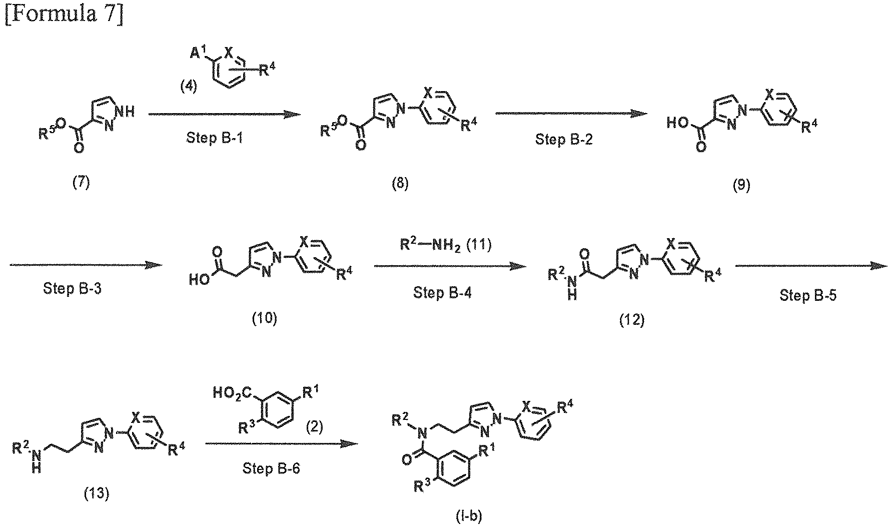

wherein, R⁵ represents a general protecting group for a carboxylic acid, e.g., the groups described in, for example, "Protective Groups in Organic Chemistry" written by J.F.W. McOmie or "Protective Groups in Organic Synthesis" written by T.W Greene and P.GM. Wuts, such as a C₁₋₆ alkyl group and a benzyl group; and other variables are as defined above.

Inventive compound (I-b) can be produced by a method shown in Scheme B.
Step B-1: Compound (8) can be prepared by a coupling reaction of Compound (7) and Compound (4). The reaction in Step B-1 can be carried out under the same reaction conditions as in Step A-2.
Step B-2: Compound (9) can be prepared by a hydrolysis reaction or a hydrogenolysis reaction of Compound (8). The reaction in Step B-2 can be carried out under reaction conditions described in, for example, "Protective Groups in Organic Chemistry" written by J.F.W. McOmie or "Protective Groups in Organic Synthesis" written by T.W. Greene and P.G.M. Wuts. For example, the reaction can be performed by hydrolysis using a mineral acid, such as hydrochloric acid or sulfuric acid, or an inorganic base, such as sodium hydroxide or potassium hydroxide, or by hydrogenolysis using a metal catalyst such as palladium or platinum in the presence of a hydrogen source. Examples of the solvent used in the reaction include alcohol solvents such as methanol and ethanol; ether solvents such as tetrahydrofuran and 1,4-dioxane; water; and mixed solvents thereof. The reaction can be carried out at 0°C to 100°C.
Step B-3: Compound (10) can be prepared by an Arndt-Eistert carbon-increasing reaction of Compound (9). The reaction in Step B-3 can be carried out under conditions for treating a carboxylic acid with, for example, thionyl chloride or oxalyl chloride in a halogen solvent such as dichloromethane and chloroform or an aromatic hydrocarbon solvent such as toluene to convert the carboxylic acid into a carboxylic acid chloride; subsequently treating the carboxylic acid chloride with, for example, diazomethane or trimethylsilyldiazomethane in acetonitrile or an ether solvent such as tetrahydrofuran and 1,4-dioxane to convert the carboxylic acid chloride into an α-diazomethyl ketone compound; and finally reacting the α-diazomethyl ketone compound with, for example, silver oxide or silver acetate in a mixed solvent of water and an ether solvent such as tetrahydrofuran and 1,4-dioxane.
Step B-4: Compound (12) can be prepared by a condensation reaction of Compound (10) and Compound (11). The reaction in Step B-4 can be carried out under the same reaction conditions as in Step A-1.
Step B-5: Compound (13) can be prepared by reduction of the amide of Compound (12). The reaction in Step B-5 can be carried out under conditions for a reaction with a reducing agent such as lithium aluminum hydride, diborane, a borane dimethyl sulfide complex, a borane tetrahydrofuran complex, sodium borohydride, lithium borohydride, sodium cyanoborohydride, or sodium triacetoxy borohydride in an alcohol solvent such as methanol or ethanol, an ether solvent such as tetrahydrofuran or 1,4-dioxane, an aromatic hydrocarbon solvent such as toluene, or a mixed solvent thereof. The reaction can be carried out at 0°C to 150°C.
Step B-6: Inventive compound (I-b) can be prepared by a condensation reaction of Compound (13) and Compound (2). The reaction in Step B-6 can be carried out under the same reaction conditions as in Step A-1.

### Scheme C

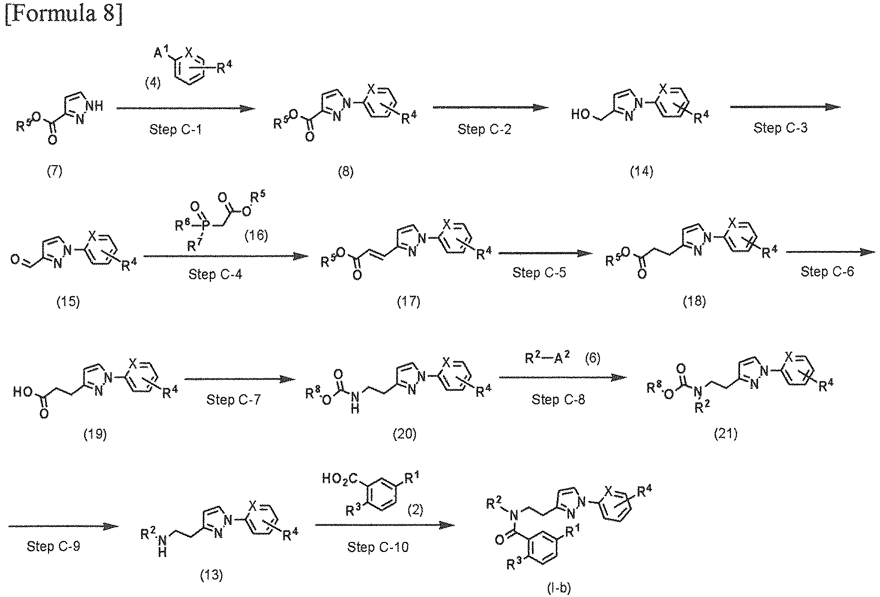

wherein, R⁶ and R⁷ each represent a C₁₋₆ alkoxy group; R⁸ represents a C₁₋₆ alkyl group or a benzyl group; and other variables are as defined above.

Inventive compound (I-b) can be also produced by another method shown in Scheme C.
Step C-1: Compound (8) can be prepared by a coupling reaction of Compound (7) and Compound (4). The reaction in Step C-1 can be carried out under the same reaction conditions as in Step A-2.
Step C-2: Compound (14) can be prepared by reduction of the ester of Compound (8). The reaction in Step C-2 can be carried out under conditions for a reaction with a reducing agent such as lithium aluminum hydride, diisobutylaluminum hydride, sodium borohydride, and lithium borohydride in an alcohol solvent such as methanol and ethanol, an ether solvent such as tetrahydrofuran and 1,4-dioxane, an aromatic hydrocarbon solvent such as toluene, or a mixed solvent thereof The reaction can be carried out at -80°C to 150°C, preferably at 0°C to 25°C.
Step C-3: Compound (15) can be prepared by oxidation of the hydroxyl group of Compound (14). The reaction in Step C-3 can be carried out under conditions for a reaction with an oxidizing agent, e.g., a hypervalent iodine compound such as a Dess-Martin reagent and 2-iodoxybenzoic acid, a chromate such as pyridinium chlorochromate and pyridinium dichromate, tetrapropylammonium perruthenate, or manganese dioxide in a halogen solvent such as dichloromethane and chloroform, dimethyl sulfoxide, or acetonitrile. The reaction can be carried out at 0°C to 150°C, preferably at 25°C to 80°C.
Step C-4: Compound (17) can be prepared by a Horner-Wadsworth-Emmons reaction of Compound (15). The reaction in Step C-4 can be carried out under conditions for treatment of a phosphonate (16) with a base such as sodium hydride, potassium hydride, tert-butoxy potassium, sodium bis(trimethylsilyl)amide, and lithium bis(trimethylsilyl)amide in a solvent, e.g., an ether solvent such as tetrahydrofuran and 1,4-dioxane, an aromatic hydrocarbon solvent such as toluene, or a mixed solvent thereof and then a reaction with an aldehyde. The reaction can be carried out at 0°C to 120°C.
Step C-5: Compound (18) can be prepared by reduction of the double bond of Compound (17). The reaction in Step C-5 can be carried out under conditions for hydrogenation in the presence of a metal catalyst such as palladium and platinum in an alcohol solvent such as methanol and ethanol, an ether solvent such as tetrahydrofuran and 1,4-dioxane, ethyl acetate, or a mixed solvent thereof. The reaction can be carried out at 25°C to 80°C.
Step C-6: Compound (19) can be prepared by hydrolysis of Compound (18). The reaction in Step C-6 can be carried out under the same reaction conditions as in Step B-2.
Step C-7: Compound (20) can be prepared from Compound (19) by a reaction through Curtius rearrangement. The reaction in Step C-7 can be carried out under conditions for converting a carboxylic acid into an isocyanate through treatment with diphenylphosphoryl azide and then heating in an aromatic hydrocarbon solvent such as toluene and then reacting the isocyanate with, for example, methanol, ethanol, tert-butyl alcohol, or benzyl alcohol.
Step C-8: Compound (21) can be prepared by alkylation of Compound (20). The reaction in Step C-8 can be carried out under the same reaction conditions as in Step A-3.
Step C-9: Compound (13) can be prepared by deprotection of Compound (21). The reaction in Step C-9 can be carried out under conditions for treatment with, for example, hydrochloric acid or trifluoroacetic acid in an alcohol solvent such as methanol and ethanol, an ether solvent such as tetrahydrofuran and 1,4-dioxane, a halogen solvent such as dichloromethane and chloroform, water, or a mixed solvent thereof. The reaction can be carried out at 0°C to 80°. Alternatively, the reaction can be carried out under conditions for hydrogenolysis using a metal catalyst such as palladium and platinum in the presence of a hydrogen source in an alcohol solvent such as methanol and ethanol, an ether solvent such as tetrahydrofuran and 1,4-dioxane, ethyl acetate, water, or a mixed solvent thereof. The reaction can be carried out at 0°C to 100°C.
Step C-10: Inventive compound (I-b) can be prepared by a condensation reaction of Compound (13) and Compound (2). The reaction in Step C-10 can be carried out under the same reaction conditions as in Step A-1.

### Scheme D

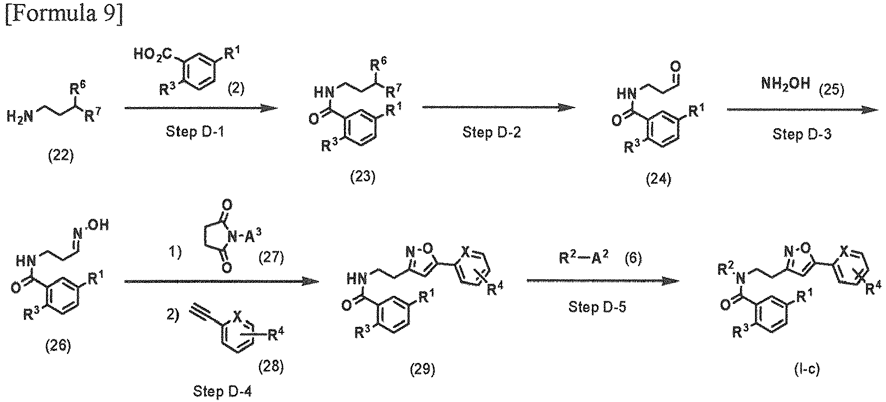

wherein, A³ represents a halogen atom; and other variables are as defined above.
Step D-1: Compound (23) can be prepared by a condensation reaction of Compound (22) and Compound (2). The reaction in Step D-1 can be carried out under the same reaction conditions as in Step A-1.
Step D-2: Compound (24) can be prepared by deacetalization of Compound (23). The reaction in Step D-2 can be carried out under conditions for a reaction with an acid such as hydrochloric acid, trifluoroacetic acid, and p-toluenesulfonic acid in an alcohol solvent such as methanol and ethanol, an ether solvent such as tetrahydrofuran and 1,4-dioxane, a halogen solvent such as dichloromethane and chloroform, acetone, water, or a mixed solvent thereof. The reaction can be carried out at 0°C to 80°C.
Step D-3: Compound (26) can be prepared by a condensation reaction of Compound (24) and hydroxylamine (25). The reaction in Step D-3 can be carried out under conditions for a reaction with hydroxylamine or its hydrochloride in a solvent in the presence or absence of a base. Examples of the base used in the reaction include organic amines such as pyridine, triethylamine, and diisopropylethylamine; inorganic bases such as sodium hydroxide, potassium hydroxide, and sodium bicarbonate; and acetates such as sodium acetate and potassium acetate. Examples of the solvent used in the reaction include alcohol solvents such as methanol and ethanol, water, and mixed solvents thereof. The reaction can be carried out at 0°C to 100°C.
Step D-4: Compound (29) can be prepared by halogenation of Compound (26) and then an isoxazole cyclization reaction. The reaction in Step D-4 can be carried out under conditions for halogenation of an oxime compound by treatment with halogenated imide Compound (27), such as N-bromosuccinimide or N-chlorosuccinimide, in an alcohol solvent such as methanol or ethanol, an ether solvent such as tetrahydrofuran or 1,4-dioxane, an aprotic polar solvent such as N,N-dimethylformamide, a halogen solvent such as dichloromethane or chloroform, an aromatic hydrocarbon solvent such as toluene, water, or acetonitrile and then a reaction with alkynylated Compound (28) in an ether solvent such as tetrahydrofuran or 1,4-dioxane in the presence of a base such as triethylamine or sodium hydroxide.
Step D-5: Inventive Compound (I-c) can be prepared by alkylation of Compound (29). The reaction in Step D-5 can be carried out under the same reaction conditions as in Step A-3.

### Scheme E

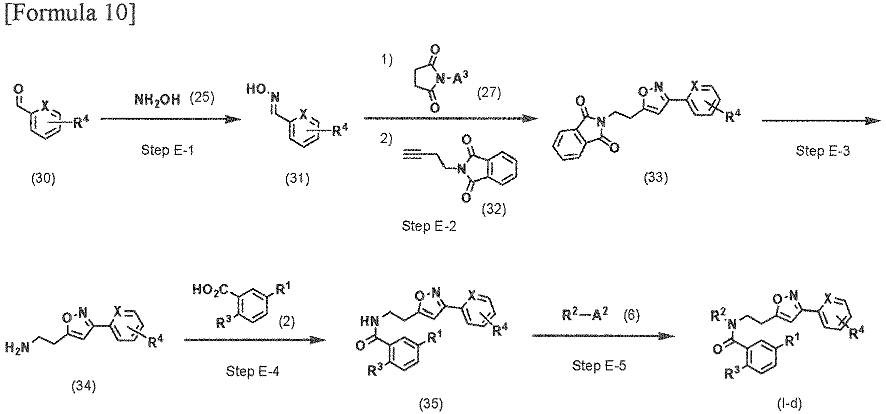

wherein, the variables are as defined above.
Step E-1: Compound (31) can be prepared by a condensation reaction of Compound (30) and hydroxylamine (25). The reaction in Step E-1 can be carried out under the same reaction conditions as in Step D-3.
Step E-2: Compound (33) can be prepared by halogenation of Compound (31) and then an isoxazole cyclization reaction. The reaction in Step E-2 can be carried out under the same reaction conditions as in Step D-4.
Step E-3: Compound (34) can be prepared by deprotection of Compound (33). The reaction in Step E-3 can be carried out under conditions for a reaction with hydrazine monohydrate in an alcohol solvent such as methanol or ethanol, an ether solvent such as tetrahydrofuran or 1,4-dioxane, a halogen solvent such as dichloromethane or chloroform, water, or a mixed solvent thereof. The reaction can be carried out at 25°C to 100°C.
Step E-4: Compound (35) can be prepared by a condensation reaction of Compound (34) and Compound (2). The reaction in Step E-4 can be carried out under the same reaction conditions as in Step A-1.
Step E-5: Inventive Compound (I-d) can be prepared by alkylation of Compound (35). The reaction in Step E-5 can be carried out under the same reaction conditions as in Step A-3.

### Scheme F

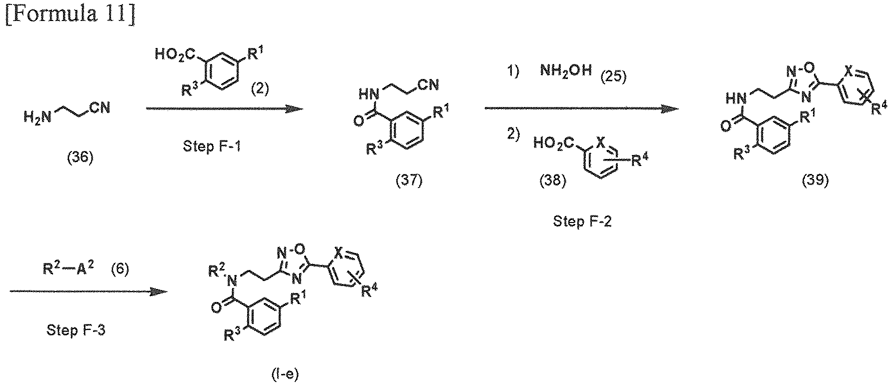

wherein, the variables are as defined above.
Step F-1: Compound (37) can be prepared by a condensation reaction of Compound (36) and Compound (2). The reaction in Step F-1 can be carried out under the same reaction conditions as in Step A-1.
Step F-2: Compound (39) can be prepared by amidoximation of Compound (37) and then an oxadiazole cyclization reaction. The reaction in Step F-2 can be carried out under conditions for treating a nitrile compound (37) with an alcohol solvent such as methanol and ethanol and hydroxylamine (25) or its hydrochloride for amidoximation and then a reaction with carboxylic acid (38) and a dehydration condensation agent such as 1-methyl-3-(3-dimethylaminopropyl)carbodiimide hydrochloride, dicyclohexylcarbodiimide, or carbonyldiimidazole in an ether solvent such as tetrahydrofuran or 1,4-dioxane, an aprotic polar solvent such as N,N-dimethylformamide, a halogen solvent such as dichloromethane or chloroform, an aromatic hydrocarbon solvent such as toluene, ethyl acetate, acetonitrile, or a mixed solvent thereof.
Step F-3: Inventive Compound (I-e) can be prepared by alkylation of Compound (39). The reaction in Step F-3 can be carried out under the same reaction conditions as in Step A-3.

### Scheme G

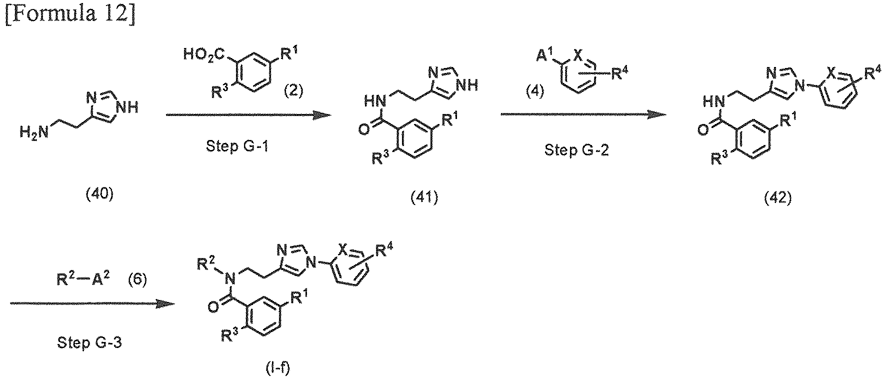

wherein, the variables are as defined above.
Step G-1: Compound (41) can be prepared by a condensation reaction of Compound (40) and Compound (2). The reaction in Step G-1 can be carried out under the same reaction conditions as in Step A-1.
Step G-2: Compound (42) can be prepared by a coupling reaction of Compound (41) and Compound (4). The reaction in Step G-2 can be carried out under the same reaction conditions as in Step A-2.
Step G-3: Inventive Compound (I-f) can be prepared by alkylation of Compound (42). The reaction in Step G-3 can be carried out under the same reaction conditions as in Step A-3.

### Scheme H

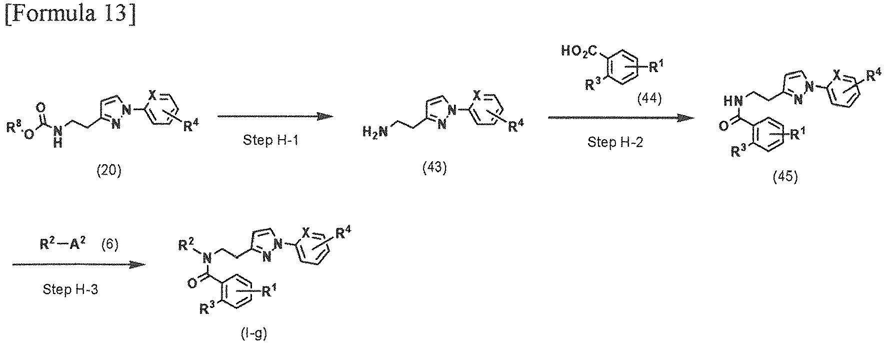

wherein, the variables are as defined above.
Step H-1: Compound (43) can be prepared by deprotection of Compound (20). The reaction in Step H-1 can be carried out under the same reaction conditions as in Step C-9.
Step H-2: Compound (45) can be prepared by a condensation reaction of Compound (43) and Compound (44). The reaction in Step H-2 can be carried out under the same reaction conditions as in Step A-1.
Step H-3: Inventive Compound (I-g) can be prepared by alkylation of Compound (45). The reaction in Step H-3 can be carried out under the same reaction conditions as in Step A-3.

### Scheme I

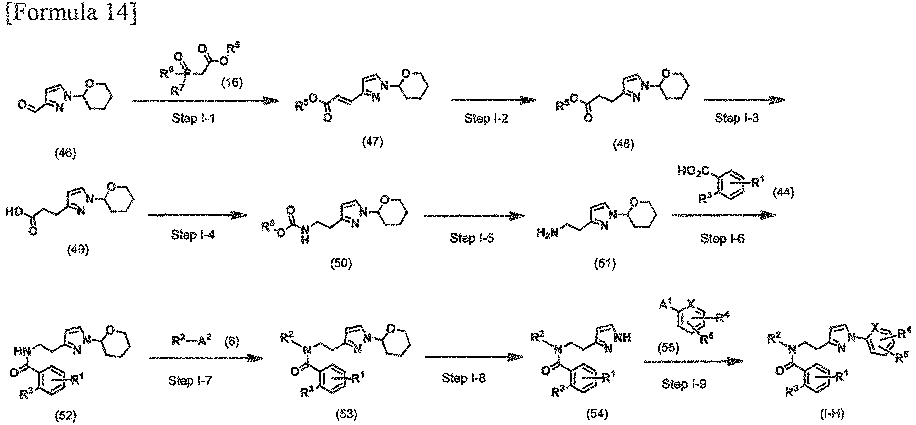

wherein, the variables are as defined above.
Step I-1: Compound (47) can be prepared by a Homer-Wadsworth-Emmons reaction of Compound (46). The reaction in Step I-1 can be carried out under the same reaction conditions as in Step C-4.
Step I-2: Compound (48) can be prepared by reduction of the double bond of Compound (47). The reaction in Step I-2 can be carried out under the same reaction conditions as in Step C-5.
Step I-3: Compound (49) can be prepared by hydrolysis of Compound (48). The reaction in Step I-3 can be carried out under the same reaction conditions as in Step B-2.
Step I-4: Compound (50) can be prepared from Compound (49) by a reaction through Curtius rearrangement. The reaction in Step I-4 can be carried out under the same reaction conditions as in Step C-7.
Step I-5: Compound (51) can be prepared by deprotection of Compound (50). The reaction in Step I-5 can be carried out under the same reaction conditions as in Step C-9.
Step I-6: Compound (52) can be prepared by a condensation reaction of Compound (51) and Compound (44). The reaction in Step I-6 can be carried out under the same reaction conditions as in Step A-1.
Step I-7: Compound (53) can be prepared by alkylation of Compound (52). The reaction in Step I-7 can be carried out under the same reaction conditions as in Step A-3.
Step I-8: Compound (54) can be prepared by deprotection of Compound (53). The reaction in Step I-8 can be carried out under conditions for treatment with, for example, hydrochloric acid or trifluoroacetic acid in an alcohol solvent such as methanol or ethanol, an ether solvent such as tetrahydrofuran or 1,4-dioxane, a halogen solvent such as dichloromethane or chloroform, water, or a mixed solvent thereof The reaction can be carried out at 0°C to 100°C.
Step I-9: Inventive Compound (I-H) can be prepared by a coupling reaction of Compound (54) and Compound (55). The reaction in Step I-9 can be carried out under the same reaction conditions as in Step A-2.

### Scheme J

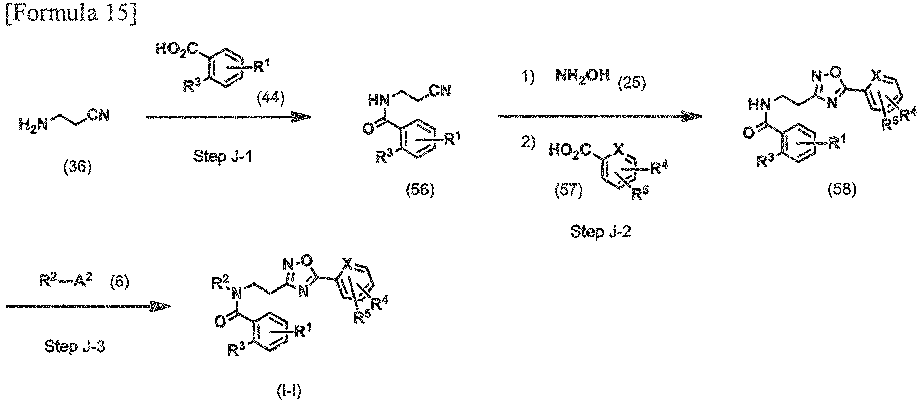

wherein, the variables are as defined above.
Step J-1: Compound (56) can be prepared by a condensation reaction of Compound (36) and Compound (44). The reaction in Step J-1 can be carried out under the same reaction conditions as in Step A-1.
Step J-2: Compound (58) can be prepared by amidoximation of Compound (56) and then an oxadiazole cyclization reaction. The reaction in Step J-2 can be carried out under the same reaction conditions as in Step F-2.
Step J-3: Inventive Compound (I-I) can be prepared by alkylation of Compound (58). The reaction in Step J-3 can be carried out under the same reaction conditions as in Step A-3.

### Scheme K

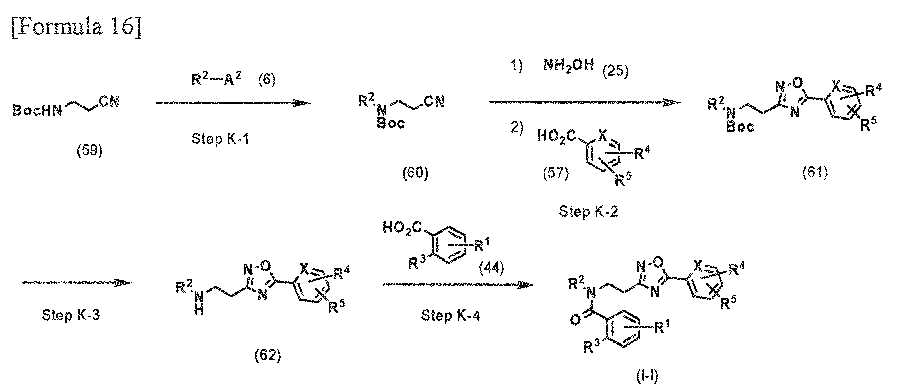

wherein, the variables are as defined above.

Inventive compound (I-I) can be also produced by another method shown in Scheme K.
Step K-1: Compound (60) can be prepared by alkylation of Compound (59). The reaction in Step K-1 can be carried out under the same reaction conditions as in Step A-3.
Step K-2: Compound (61) can be prepared by amidoximation of Compound (60) and then an oxadiazole cyclization reaction. The reaction in Step K-2 can be carried out under the same reaction conditions as in Step F-2.
Step K-3: Compound (62) can be prepared by deprotection of Compound (61). The reaction in Step K-3 can be carried out under the same reaction conditions as deprotection under an acidic condition in Step C-9.
Step K-4: Inventive Compound (I-I) can be prepared by a condensation reaction of Compound (62) and Compound (44). The reaction in Step K-4 can be carried out under the same reaction conditions as in Step A-1.

### Scheme L

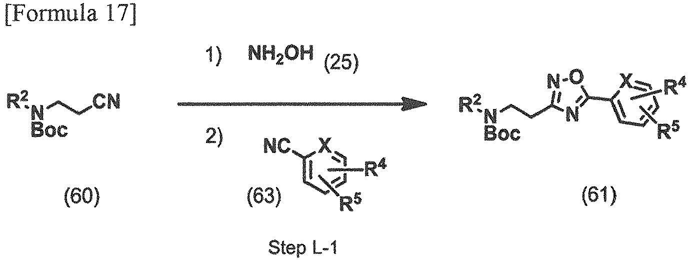

wherein, the variables are as defined above.

Compound (61) can also be produced by another method shown in Scheme L.
Step L-1: Compound (61) can be prepared by amidoximation of Compound (60) and then an oxadiazole cyclization reaction. The reaction in Step L-1 can be carried out under conditions for treating a nitrile compound (60) with an alcohol solvent such as methanol or ethanol and hydroxylamine (25) or its hydrochloride for amidoximation and then a reaction with benzonitrile (63) in the presence of p-toluenesulfonic acid monohydrate and zinc chloride in an aprotic polar solvent such as N,N-dimethylformamide. The reaction can be carried out at 80°C to 120°C.

### [Examples]

The present invention will now be described in more detail by Reference Examples, Examples, and Test Example, which do not limit the present invention and may be modified within the scope of the invention.

In the following Reference Examples and Examples, "KP-Sil", "HP-Sil", and "KP-NH" used for purification by column chromatography were respectively SNAPCartridge KP-Sil, SNAPCartridge HP-Sil, and SNAPCartridge KP-NH manufactured by Biotage, Inc.

In the following Reference Examples and Examples, "ISOLUTE Phase Separator" used for aftertreatment operation was ISOLUTE Phase Separator manufactured by Biotage, Inc.

In the following Reference Examples and Examples, purification by preparative high-performance liquid chromatography (HPLC) was carried out under the following two different conditions, provided that when a trifluoroacetic acid was used in this process for a compound having a basic functional group, a process, such as neutralization, for preparing a free form was performed in some cases.

### Condition 1

Instrument: Gilson, preparative HPLC system
Column: Shiseido, Capcell Pak C18 MGII, 5 µm, 20 × 150 mm
Solvent: liquid A; water containing 0.1% trifluoroacetic acid, liquid B; acetonitrile containing 0.1% trifluoroacetic acid
Gradient: 0 min (liquid A/liquid B = 90/10), 22 min (liquid A/liquid B = 20/80), 25 min (liquid A/liquid B = 10/90)
Flow rate: 20 mL/min, Detection: UV 254 nm

### Condition 2

Instrument: Gilson, Trilution LC
Column: Waters, SunFire prep C18 OBD, 5.0 µm, 30 × 50 mm or YMC YMC-Actus Triant C18, 5.0 µm, 30 × 50 mm
Solvent: liquid A; water containing 0.1% trifluoroacetic acid, liquid B; acetonitrile containing 0.1% trifluoroacetic acid
Gradient: 0 min (liquid A/liquid B = 90/10), 11 min (liquid A/liquid B = 20/80), 12 to 13.5 min (liquid A/liquid B = 5/95)
Flow rate: 40 mL/min, Detection: UV 254 nm

In the following Reference Examples and Examples, high-performance liquid chromatography-mass spectrometry (LCMS) was performed under any of the following three different conditions.

### Condition 1

Measuring instrument: MicroMass, Platform LC and Agilent Agilent 1100
Column: Waters, SunFire C18, 2.5 µm, 4.6 mm I.D. x 50 mm
Solvent: liquid A; water containing 0.1% trifluoroacetic acid, liquid B; acetonitrile containing 0.1% trifluoroacetic acid
Gradient: 0 min (liquid A/liquid B = 90/10), 0.5 min (liquid A/liquid B = 90/10), 5.5 min (liquid A/liquid B = 20/80), 6.0 min (liquid A/liquid B = 1/99), 6.3 min (liquid A/liquid B = 1/99)
Flow rate: 1 mL/min, Detection: UV 254 nm
Ionization method: electron spray ionization (ESI)

### Condition 2

Measuring instrument: SHIMADZU, LCMS-2010EV
Column: SHIMADZU, Shim-pack XR-ODS, 2.2 µm, 2.0 mm I.D. x 30 mm
Solvent: liquid A; water containing 0.1% formic acid, liquid B; acetonitrile containing 0.1% formic acid
Gradient: 0 min (liquid A/liquid B = 90/10), 1 min (liquid A/liquid B = 60/40), 2 min (liquid A/liquid B = 0/100), 2.5 min (liquid A/liquid B = 0/100)
Flow rate: 0.6 mL/min, Detection: UV 254 nm
Ionization method: electron spray ionization (ESI) and atmospheric pressure chemical ionization (APCI)

### Condition 3

Measuring instrument: Agilent, Agilent 2900 and Agilent 6150
Column: Waters, Acquity CSH C18, 1.7 µm, 2.1 × 50 mm
Solvent: liquid A; water containing 0.1% formic acid, liquid B; acetonitrile containing 0.1% formic acid
Gradient: 0 min (liquid A/liquid B = 80/20), 1.2 to 1.4 min (liquid A/liquid B = 1/99)
Flow rate: 0.8 mL/min, Detection: UV 254 nm
Ionization method: electron spray ionization (ESI)

In the following Reference Examples and Examples, each mass spectrum (MS) was measured under the following condition.

MS measuring instrument: SHIMADZU LCMS-2010EV or Micromass, Platform LC

In the following Reference Examples and Examples, compounds were named using ACD/Name (ACD/Labs 12.01, Advanced Chemistry Development Inc.).

In Reference Examples and Examples, terms and reagents are denoted by the following abbreviations:
Na₂SO₄ (anhydrous sodium sulfate), NaHCO₃ (sodium bicarbonate), NaOH (sodium hydroxide), NH₄Cl (ammonium chloride), MeOH (methanol), EtOH (ethanol), Et₂O (diethyl ether), THF (tetrahydrofuran), DMF (N,N-dimethylformamide), MeCN (acetonitrile), EtOAc (ethyl acetate), CHCl₃ (chloroform), HOBt·H₂O (1-hydroxybenzotriazole monohydrate), EDC·HCl [1-(3-dimethylaminopropyl)-3-ethylcarbodiimide monohydrochloride], HATU [O-(7-azabenzotriazol-1-yl)-N,N,N',N'-tetramethyluronium hexafluorophosphate], brine (saturated brine), Boc (tert-butoxycarbonyl), Et₃N (triethylamine), DIPEA (N,N-diisopropylethylamine), MeI (methyl iodide), EtI (ethyl iodide), NaH (sodium hydride), HCl (hydrogen chloride), DIBAL-H (diisobutylaluminum hydride), and MnO₂ (manganese dioxide).

### Reference Example 1: 5-Methyl-N-[2-(1H-pyrazol-4-yl)ethyl]-2-(2H-1,2,3-triazol-2-yl)benzamide

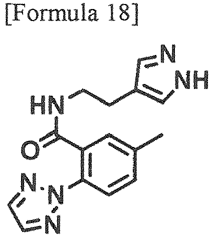

Et₃N (0.45 mL, 3.3 mmol) was added to a solution of 2-(1H-pyrazol-4-yl)ethanamine dihydrochloride (0.30 g, 1.6 mmol) in DMF (5 mL) at room temperature, followed by stirring. 5-Methyl-2-(2H-1,2,3-triazol-2-yl)benzoic acid (0.36 g, 1.8 mmol), HOBt·H₂O (0.37 g, 2.4 mmol), and EDC·HCl (0.47 g, 2.4 mmol) were added to the reaction solution, followed by stirring at room temperature for 3 hours. An aqueous NaHCO₃ solution was added to the reaction mixture, followed by extraction with EtOAc. The organic layer was washed with water and brine and was dried with Na₂SO₄. The desiccant was then removed by filtration, and the solvent was distilled off under reduced pressure. The resulting residue was purified by column chromatography (KP-NH: 28 g, CHCl₃/MeOH = 100/0 to 97/3) to give the titled compound (0.43 g, colorless and amorphous).
MS (ESI pos.) m/z: 297 [M+H]+

### Reference Example 2: N-[2-(1H-Pyrazol-4-yl)ethyl]-2-(2H-1,2,3-triazol-2-yl)benzamide

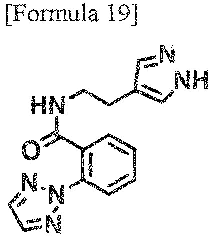

The titled compound (0.34 g, colorless and amorphous) was prepared in the same manner as in Reference Example 1 using 2-(1H-pyrazol-4-yl)ethanamine dihydrochloride (0.30 g, 1.6 mmol) and 2-(2H-1,2,3-triazol-2-yl)benzoic acid (0.34 g, 1.8 mmol) as raw materials. MS (ESI pos.) m/z: 283 [M+H]+

### Reference Example 3: N-{2-[1-(5-Fluoropyridin-2-yl)-1H-pyrazol-4-yl]ethyl}-5-methyl-2-(2H-1,2,3-triazol-2-yl)benzamide

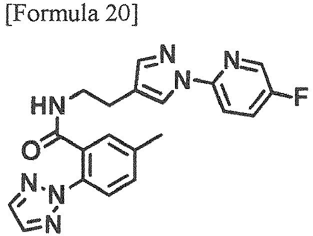

Copper(I) iodide (0.13 g, 0.4 mmol), rac-trans-N,N-dimethylcyclohexane-1,2-diamine (0.25 g, 1.5 mmol), and cesium carbonate (0.99 g, 3.1 mmol) were added to a solution of 5-methyl-N-[2-(1H-pyrazol-4-yl)ethyl]-2-(2H-1,2,3-triazol-2-yl)benzamide (0.43 g, 1.5 mmol) prepared in Reference Example 1 and 2-bromo-5-fluoropyridine (0.31 g, 1.7 mmol) in DMF (1.5 mL), followed by stirring at 80°C for 15 hours. After cooling to room temperature, brine was added to the reaction mixture, followed by extraction with EtOAc. The organic layer was washed with water and brine and was dried with Na₂SO₄. The desiccant was then removed by filtration, and the solvent was distilled off under reduced pressure. The resulting residue was purified by column chromatography (HP-Sil: 50 g, hexane/EtOAc = 70/30 to 0/100 to CHCl₃/MeOH = 80/20). The resulting solid was washed with EtOAc with stirring and was then collected by filtration to give the titled compound (0.14 g, brown solid).
MS (ESI pos.) m/z: 392 [M+H]+

### Reference Example 4: N-{2-[1-(5-Fluoropyridin-2-yl)-1H-pyrazol-4-yl]ethyl}-2-(2H-1,2,3-triazol-2-yl)benzamide

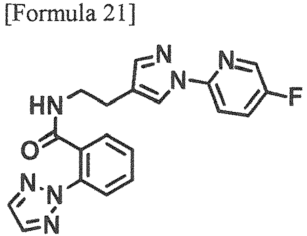

The titled compound (0.053 g, colorless solid) was prepared in the same manner as in Reference Example 3 using N-[2-(1H-pyrazol-4-yl)ethyl]-2-(2H-1,2,3-triazol-2-yl)benzamide (0.34 g, 1.2 mmol) prepared in Reference Example 2 and 2-bromo-5-fluoropyridine (0.30 g, 1.7 mmol) as raw materials.
MS (ESI pos.) m/z: 378 [M+H]+

### Reference Example 5: Ethyl-1-(5-fluoropyridin-2-yl)-1H-pyrazole-3-carboxylate

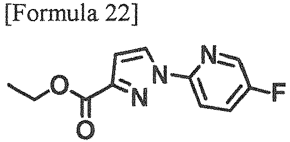

The titled compound (21.1 g, colorless solid) was prepared in the same manner as in Reference Example 3 using ethyl-1H-pyrazole-3-carboxylate (18.3 g, 130.4 mmol) and 2-bromo-5-fluoropyridine (16.1 mL, 156.4 mmol) as raw materials.
MS (ESI pos.) m/z: 236 [M+H]+

### Reference Example 6: 1-(5-Fluoropyridin-2-yl)-1H-pyrazol-3-carboxylic acid

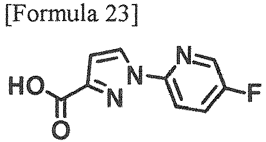

NaOH (3.52 g, 88.0 mmol) was added to a solution of ethyl-1-(5-fluoropyridin-2-yl)-1H-pyrazole-3-carboxylate (10.4 g, 44.0 mmol) prepared in Reference Example 5 in a mixture of EtOH (52 mL) and H₂O (52 mL), followed by stirring at 90°C for 2 hours. After cooling at room temperature, the solvent was distilled off under reduced pressure. An aqueous 1 mol/L HCl solution was added to the resulting residue under ice cooling. The precipitated solid was collected by filtration, washed, and dried to give the titled compound (5.96 g, colorless solid).
MS (ESI pos.) m/z: 206 [M-H]-

### Reference Example 7: [1-(5-Fluoropyridin-2-yl)-1H-pyrazol-3-yl]acetic acid

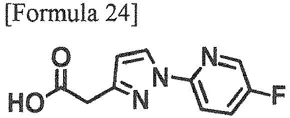

Oxalyl chloride (2.3 mL, 26.8 mmol) and DMF (5 drops) were added to a solution of 1-(5-fluoropyridin-2-yl)-1H-pyrazol-3-carboxylic acid (2.79 g, 13.4 mmol) prepared in Reference Example 6 in CHCl₃ (56 mL), followed by stirring at room temperature for 1 hour. The solvent was distilled off under reduced pressure. The residue was then dissolved in THF (28 mL) and CH₃CN (28 mL), and trimethylsilyldiazomethane (2 mol/L solution in diethyl ether, 13.4 mL, 26.8 mmol) was added thereto under ice cooling. The mixture was warmed to room temperature and was then stirred for 1 hour. The solvent was distilled off under reduced pressure. The residue was then dissolved in 1,4-dioxane (42 mL) and water (42 mL), and silver acetate (0.67 g, 4.03 mmol) was added thereto. The mixture was stirred at 130°C for 11 hours. After cooling at room temperature, the mixture was filtered through Celite (registered trademark). The filtrate was concentrated under reduced pressure. EtOAc and an aqueous NaHCO₃ solution were added to the resulting residue, and the aqueous layer was collected. An aqueous 1 mol/L HCl solution was added to the aqueous layer, followed by extraction with CHCl₃, distillation of the solvent under reduced pressure, and drying to give the titled compound (0.84 g, yellow solid).
MS (ESI pos.) m/z: 220 [M-H]-

### Reference Example 8: N-Ethyl-2-[1-(5-fluoropyridin-2-yl)-1H-pyrazol-3-yl]acetamide

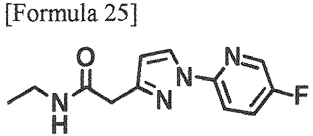

The titled compound (0.26 g, yellow solid) was prepared in the same manner as in Reference Example 1 using [1-(5-fluoropyridin-2-yl)-1H-pyrazol-3-yl]acetic acid (0.39 g, 1.78 mmol) prepared in Reference Example 7 and ethylamine (2 mol/L solution in THF, 1.8 mL, 3.6 mmol) as raw materials.
MS (ESI pos.) m/z: 249 [M+H]+

### Reference Example 9: N-Methyl-2-[1-(5-fluoropyridin-2-yl)-1H-pyrazol-3-yl]acetamide

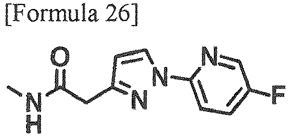

The titled compound (0.27 g, yellow solid) was prepared in the same manner as in Reference Example 1 using [1-(5-fluoropyridin-2-yl)-1H-pyrazol-3-yl]acetic acid (0.42 g, 1.90 mmol) prepared in Reference Example 7 and methylamine (2 mol/L solution in THF, 1.9 mL, 3.80 mmol) as raw materials.
MS (ESI pos.) m/z: 235 [M+H]+

### Reference Example 10: N-Ethyl-2-[1-(5-fluoropyridin-2-yl)-1H-pyrazol-3-yl]ethanamine

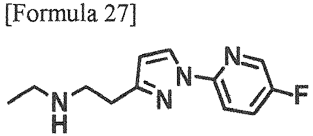

A borane tetrahydrofuran complex (1.06 mol/L solution in THF, 8.7 mL, 9.17 mmol) was added to a solution of N-ethyl-2-[1-(5-fluoropyridin-2-yl)-1H-pyrazol-3-yl]acetamide (1.13 g, 4.59 mmol) prepared in Reference Example 8 in THF (11.4 mL), followed by stirring at 70°C for 2 hours. An aqueous 2 mol/L HCl solution was then added thereto, followed by stirring at 70°C for 1 hour. After cooling at room temperature, chloroform and an aqueous HCl solution were added to the mixture, and the aqueous layer was collected. An aqueous sodium hydroxide solution was added to the aqueous layer, followed by extraction with chloroform. The solvent was distilled off under reduced pressure. The resulting residue was purified by HPLC (Condition 1) to give the titled compound (0.27 g, colorless oil). MS (ESI pos.) m/z: 235 [M+H]+

### Reference Example 11: N-Methyl-2-[1-(5-fluoropyridin-2-yl)-1H-pyrazol-3-yl]ethanamine

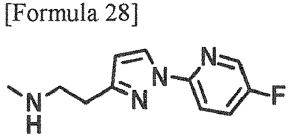

The titled compound (0.13 g, colorless oil) was prepared in the same manner as in Reference Example 10 using N-methyl-2-[1-(5-fluoropyridin-2-yl)-1H-pyrazol-3-yl]acetamide (1.04 g, 4.43 mmol) prepared in Reference Example 9 as a raw material.
MS (ESI pos.) m/z: 221 [M+H]+

### Reference Example 12: [1-(5-Fluoropyridin-2-yl)-1H-pyrazol-3-yl]methanol

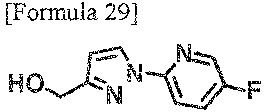

DIBAL-H (1.02 mol/L solution in hexane, 137 mL, 139 mmol) was added to a solution of ethyl-1-(5-fluoropyridin-2-yl)-1H-pyrazole-3-carboxylate (14.9 g, 64.3 mmol) prepared in Reference Example 5 in THF (150 mL) under ice cooling, followed by stirring under ice cooling for 1.5 hours. An aqueous 2 mol/L HCl solution (45 mL) was added to the reaction mixture under ice cooling, followed by stirring at the same temperature for 25 min. The THF and the hexane were distilled under reduced pressure. MeOH (75 mL), CHCl₃ (150 mL), and an aqueous 2 mol/L HCl solution (250 mL) were added to the residue under ice cooling, followed by stirring at the same temperature for 20 min and at room temperature for 50 min. An aqueous 2 mol/L NaOH solution (300 mL) and potassium sodium tartrate (Rochelle salt) (39.3 g, 139 mmol) were added to the mixture under ice cooling, followed by stirring at room temperature for 20 min. The mixture was separated into an organic layer and an aqueous layer, and the aqueous layer was extracted with CHCl₃ (150 mL)/MeOH (75 mL). Rochelle salt (39.3 g, 139 mmol) was added to the aqueous layer, followed by stirring at room temperature for 2 hours and then extraction with CHCl₃ (150 mL)/MeOH (75 mL) three times. The organic layers were combined and allowed to pass through ISOLUTE Phase Separator. The filtrate was concentrated under reduced pressure. The resulting residue was purified by column chromatography (HP-Sil: 340 g, CHCl₃/MeOH = 100/0 to 95/5) to give the titled compound (11.6 g, colorless solid).
MS (ESI/APCI pos.) m/z: 194 [M+H]+

### Reference Example 13: 1-(5-Fluoropyridin-2-yl)-1H-pyrazole-3-carbaldehyde

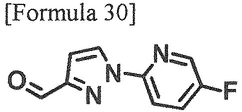

Under ice cooling, 85% MnO₂ (6.04 g, 59.0 mmol) was added to a suspension of [1-(5-fluoropyridin-2-yl)-1H-pyrazol-3-yl]methanol (0.76 g, 3.93 mmol) prepared in Reference Example 12 in CHCl₃ (15 mL), followed by stirring at room temperature for 1.5 hours and then at an oil bath temperature of 50°C for 45 min. CHCl₃ (7.6 mL) and 85% MnO₂ (10.1 g, 98.4 mmol) were added to the reaction mixture, followed by stirring at room temperature for 45 min. CHCl₃ (15 mL) and 85% MnO₂ (4.02 g, 39.3 mmol) were added to the reaction mixture, followed by stirring at room temperature for 1 hour. The reaction mixture was filtered through Celite (registered trademark), the solid was washed with CHCl₃, and then the filtrate was concentrated under reduced pressure. The resulting residue was purified by column chromatography (HP-Sil: 25 g, hexane/CHCl₃ = 100/0 to 20/80, HP-Sil: 10 g, hexane/EtOAc = 100/0 to 80/20) to give the titled compound (0.133 g, colorless solid).
MS (ESI pos.) m/z: 192 [M+H]+

### Reference Example 14: (E)-Ethyl-3-[1-(5-fluoropyridin-2-yl)-1H-pyrazol-3-yl]acrylate

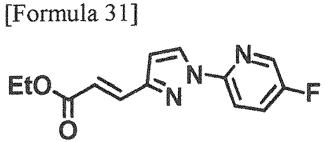

Ethyl-2-(diethoxyphosphoryl)acetate (0.163 mL, 0.816 mmol) was added to a suspension of 60% NaH (0.033 g, 0.82 mmol) in THF (1.3 mL) under ice cooling, followed by stirring at the same temperature for 5 min. This mixture and THF (0.65 mL) were added to a suspension of 1-(5-fluoropyridin-2-yl)-1H-pyrazole-3-carbaldehyde (0.130 g, 0.680 mmol) prepared in Reference Example 13 in THF (1.3 mL) under ice cooling, followed by stirring at room temperature for 1.5 hours. A saturated aqueous NH₄Cl solution (0.65 mL), water (2.6 mL), and CHCl₃ (3.3 mL) were added to the reaction mixture, and the mixture was separated into an organic layer and an aqueous layer. The aqueous layer was extracted with CHCl₃ twice. The organic layers were combined and allowed to pass through ISOLUTE Phase Separator. The filtrate was concentrated under reduced pressure. The resulting residue was purified by column chromatography (HP-Sil: 10 g, CHCl₃/MeOH = 100/0) to give the titled compound (0.177 g, colorless solid).
MS (ESI pos.) m/z: 262 [M+H]+

### Reference Example 15: 3-[1-(5-Fluoropyridin-2-yl)-1H-pyrazol-3-yl]propanoic acid

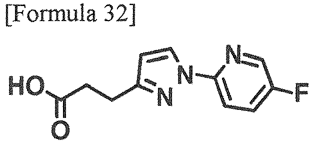

A suspension of (E)-ethyl-3-[1-(5-fluoropyridin-2-yl)-1H-pyrazol-3-yl]acrylate (0.177 g, 0.678 mmol) prepared in Reference Example 14 and Pd/C 5% (0.144 g, 0.0678 mmol) in EtOH (3.5 mL) was stirred under a hydrogen atmosphere at room temperature for 17 hours. The reaction mixture was filtered through Celite (registered trademark). The solid was washed with EtOH, CHCl₃, and CHCl₃/MeOH = 1/1 in this order, and the filtrate was concentrated under reduced pressure. The resulting residue was suspended in EtOH (3.4 mL). Water (0.650 mL) and an aqueous 2 mol/L NaOH solution (0.650 mL, 1.30 mmol) were added to the suspension, followed by stirring at 70°C for 2.5 hours. The reaction mixture was cooled to room temperature. EtOH was distilled under reduced pressure, and water (3.4 mL) and Et₂O (3.4 mL) were added to the residue. The mixture was separated into an organic layer and an aqueous layer. Water (0.98 mL), an aqueous 2 mol/L HCl solution (0.98 mL, 1.95 mmol), CHCl₃ (3.4 mL), and MeOH (1.7 mL) were added to the aqueous layer. The mixture was separated into an organic layer and an aqueous layer, and the aqueous layer was extracted with CHCl₃ (3.4 mL) twice. The organic layers were combined and were dried with Na₂SO₄. The desiccant was removed by filtration. The filtrate was then washed with CHCl₃/MeOH = 2/1 and was concentrated under reduced pressure to give the titled compound (0.141 g, colorless solid).
MS (ESI/APCI pos.) m/z: 258 [M+Na]+

### Reference Example 16: Tert-Butyl{2-[1-(5-fluoropyridin-2-yl)-1H-pyrazol-3-yl]ethyl} carbamate

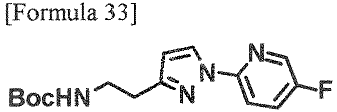

Diphenylphosphoryl azide (0.155 mL, 0.719 mmol) and Et₃N (0.1 mL, 0.719 mmol) were added to a suspension of 3-[1-(5-fluoropyridin-2-yl)-1H-pyrazol-3-yl]propanoic acid (0.141 g, 0.599 mmol) prepared in Reference Example 15 in tert-butyl alcohol (2.8 mL), followed by heating under reflux for 5 hours. The reaction mixture was cooled to room temperature, and CHCl₃ (8.5 mL), saturated aqueous NaHCO₃ solution (1.4 mL), water (4.2 mL), and MeOH (4.2 mL) were added to the mixture, followed by separation into an organic layer and an aqueous layer. The aqueous layer was extracted with CHCl₃ (2.8 mL)/MeOH (1.4 mL) and then with CHCl₃ (2.8 mL). The organic layers were combined and allowed to pass through ISOLUTE Phase Separator. The filtrate was concentrated under reduced pressure. The resulting residue was purified by column chromatography (HP-Sil: 10 g, CHCl₃/MeOH = 100/0 to 97/3, KP-NH: 11 g, hexane/EtOAc = 100/0 to 90/10, HP-Sil: 10 g, hexane/EtOAc = 100/0 to 80/20) to give the titled compound (0.088 g, colorless solid).
MS (ESI/APCI pos.) m/z: 329 [M+Na]+

### Reference Example 17: Tert-Butyl ethyl{2-[1-(5-fluoropyridin-2-yl)-1H-pyrazol-3-yl]ethyl carbamate

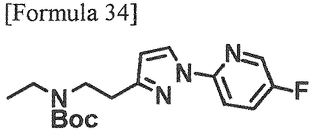

EtI (0.028 mL, 0.34 mmol) and 60% NaH (0.014 g, 0.34 mmol) were added to a solution of tert-butyl{2-[1-(5-fluoropyridin-2-yl)-1H-pyrazol-3-yl]ethyl}carbamate (0.088 g, 0.29 mmol) prepared in Reference Example 16 in THF (1.8 mL), followed by stirring at room temperature for 3 hours. EtI (0.028 mL, 0.34 mmol) and 60% NaH (0.014 g, 0.34 mmol) were added to the reaction mixture, followed by stirring at room temperature for 3 days. A saturated aqueous NH₄Cl solution (0.88 mL), water (0.88 mL), and CHCl₃ (1.8 mL) were added to the reaction mixture, followed by separation into an organic layer and an aqueous layer. The aqueous layer was extracted with CHCl₃ (1.8 mL) twice. The organic layers were combined and allowed to pass through ISOLUTE Phase Separator. The filtrate was then concentrated under reduced pressure. The resulting residue was purified by column chromatography (HP-Sil: 10 g, hexane/EtOAc = 100/0 to 85/15) to give the titled compound (0.069 g, colorless oil).
MS (ESI/APCI pos.) m/z: 335 [M+H]+

### Reference Example 18: N-Ethyl-2-[1-(5-fluoropyridin-2-yl)-1H-pyrazol-3-yl]ethanamine hydrochloride

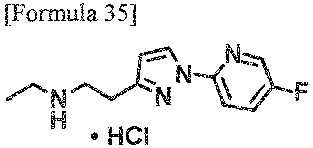

A 4 mol/L HCl-EtOAc solution (1.0 mL, 4.0 mmol) was added to a solution of tert-butyl ethyl{2-[1-(5-fluoropyridin-2-yl)-1H-pyrazol-3-yl]ethyl}carbamate (0.069 g, 0.21 mmol) prepared in Reference Example 17 in EtOAc (0.69 mL), followed by stirring at room temperature for 1.5 hours. The reaction mixture was concentrated under reduced pressure to give the titled compound (0.056 g, colorless solid).
MS (ESI/APCI pos.) m/z: 235 [M+H]+

### Reference Example 19: N-(3,3-Diethoxypropyl)-5-methyl-2-(2H-1,2,3-triazol-2-yl)benzamide

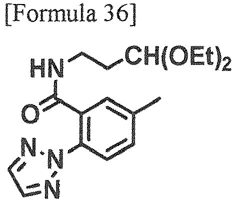

HOBt·H₂O (1.1 g, 7.4 mmol) and EDC·HCl (1.4 g, 7.4 mmol) were added to a solution of 3,3-diethoxypropan-1-amine (0.96 mL, 5.9 mmol) and 5-methyl-2-(2H-1,2,3-triazol-2-yl)benzoic acid (1.0 g, 4.9 mmol) in DMF (18 mL), followed by stirring at room temperature for 3 hours. An aqueous NaHCO₃ solution was added to the reaction solution, followed by extraction with EtOAc. The organic layer was washed with water and brine and was dried with Na₂SO₄. The desiccant was then removed by filtration, and the solvent was distilled off under reduced pressure. The resulting residue was purified by column chromatography (KP-Sil: 25 g, hexane/EtOAc = 85/15 to 0/100) to give the titled compound (1.6 g, colorless solid).
MS (ESI pos.) m/z: 333 [M+H]+

### Reference Example 20: N-[(3E)-3-(Hydroxyimino)propyl]-5-methyl-2-(2H-1,2,3-triazol-2-yl)benzamide

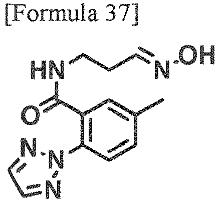

An aqueous 2 mol/L HCl solution was added to a solution of N-(3,3-diethoxypropyl)-5-methyl-2-(2H-1,2,3-triazol-2-yl)benzamide (0.88 g, 2.7 mmol) prepared in Reference Example 19 in THF (6 mL), followed by stirring at room temperature for 1 hour. An aqueous NaHCO₃ solution was added to the reaction solution to adjust the pH to a neutral level, followed by extraction with EtOAc. The organic layer was washed with brine and was dried with Na₂SO₄. The desiccant was then removed by filtration, and the solvent was distilled off under reduced pressure. Sodium acetate (0.31 g, 3.7 mmol) and hydroxylamine hydrochloride (0.26 g, 3.7 mmol) were added to an aqueous solution of the residue in 95% EtOH (7.5 mL), followed by stirring in an ice bath for 2 hours. An aqueous NaHCO₃ solution was added to the reaction solution, followed by extraction with EtOAc. The organic layer was washed with brine and was dried with Na₂SO₄. The desiccant was then removed by filtration, and the solvent was distilled off under reduced pressure. The resulting solid was washed with EtOAc with stirring and was then collected by filtration to give the titled compound (0.45 g, colorless solid).
MS (ESI pos.) m/z: 274 [M+H]+

### Reference Example 21: N-{2-[5-(5-Fluoropyridin-2-yl)-1,2-oxazol-3-yl]ethyl}-5-methyl-2-(2H-1,2,3-triazal-2-yl)benzamide

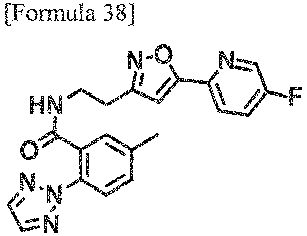

N-chlorosuccinimide (0.24 g, 1.8 mmol) was added to a solution of N-[(3E)-3-(hydroxyimino)propyl]-5-methyl-2-(2H-1,2,3-triazol-2-yl)benzamide (0.45 g, 1.6 mmol) prepared in Reference Example 20 in DMF (5 mL) in an ice bath. The mixture was warmed to room temperature, followed by stirring for 15 hours. Water was added to the reaction solution, followed by extraction with EtOAc. The organic layer was washed with water and brine and was dried with Na₂SO₄. The desiccant was then removed by filtration, and the solvent was distilled off under reduced pressure. The residue was dissolved in THF (3.3 mL), and a solution of 2-ethynyl-5-fluoropyridine (0.30 g, 2.5 mmol) in THF (1 mL) was dropwise added to the solution in an ice bath. Et₃N (0.30 mL, 2.2 mmol) was further dropwise added thereto. The mixture was warmed to room temperature, followed by stirring for 15 hours. Water was added to the reaction solution, followed by extraction with CHCl₃. The organic layer was washed with brine and was washed with Na₂SO₄. The desiccant was then removed by filtration, and the solvent was distilled off under reduced pressure. The resulting residue was purified by column chromatography (HP-Sil: 50 g, CHCl₃/MeOH = 99/1 to 95/5). The resulting solid was washed with EtOAc with stirring and was then collected by filtration to give the titled compound (0.27 g, colorless solid).
MS (ESI pos.) m/z: 393 [M+H]+

### Reference Example 22: (E)-1-(5-Fluoropyridin-2-yl)-N-hydroxymethanimine

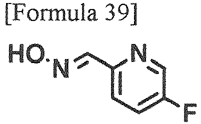

Sodium acetate (0.1 g, 1.2 mmol) and hydroxylamine hydrochloride (0.085 g, 1.2 mmol) were added to an aqueous solution of 5-fluoropyridine-2-carbaldehyde (0.10 g, 0.82 mmol) in 95% EtOH (2.5 mL) under ice cooling, followed by stirring for 2 hours. An aqueous NaHCO₃ solution was added to the reaction solution, followed by extraction with EtOAc. The organic layer was washed with brine and was dried with Na₂SO₄. The desiccant was then removed by filtration, and the solvent was distilled off under reduced pressure. The resulting residue was purified by column chromatography (KP-NH: 11 g, hexane/EtOAc = 75/25 to 0/100) to give the titled compound (0.054 g, yellow solid).
MS (ESI pos.) m/z: 141 [M+H]+

### Reference Example 23: 2-{2-[3-(5-Fluoropyridin-2-yl)-1,2-oxazol-5-yl]ethyl}-1H-isoindole-1,3(2H)-dione

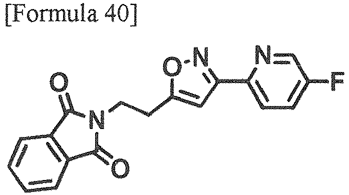

The titled compound (0.15 g, colorless solid) was prepared in the same manner as in Reference Example 21 using (E)-1-(5-fluoropyridin-2-yl)-N-hydroxymethanimine (0.29 g, 2.0 mmol) prepared in Reference Example 22 and 2-(but-3-yn-1-yl)-1H-isoindole-1,3(2H)-dione (0.31 g, 1.6 mmol) as raw materials.
MS (ESI pos.) m/z: 338 [M+H]+

### Reference Example 24: 2-[3-(5-Fluoropyridin-2-yl)-1,2-oxazol-5-yl]ethanamine

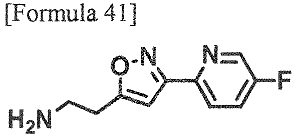

Hydrazine monohydrate (0.043 mL, 0.88 mmol) was dropwise added to a solution of 2-{2-[3-(5-fluoropyridin-2-yl)-1,2-oxazol-5-yl]ethyl}-1H-isoindole-1,3(2H)-dione (0.15 g, 0.44 mmol) prepared in Reference Example 23 in EtOH (1.2 mL), followed by stirring at 90°C for 2 hours. After cooling at room temperature, the precipitated solid was removed by filtration. The filtrate was concentrated under reduced pressure to give the titled compound (0.094 g, colorless and amorphous).
MS (ESI pos.) m/z: 208 [M+H]+

### Reference Example 25: N-{2-[3-(5-Fluoropyridin-2-yl)-1,2-oxazol-5-yl]ethyl}-5-methyl-2-(2H-1,2,3-triazol-2-yl)benzamide

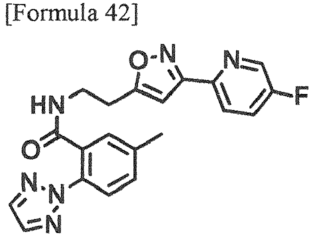

The titled compound (0.040 g, colorless solid) was prepared in the same manner as in Reference Example 19 using 2-[3-(5-fluoropyridin-2-yl)-1,2-oxazol-5-yl]ethanamine (0.46 g, 0.22 mmol) prepared in Reference Example 24 and 5-methyl-2-(2H-1,2,3-triazol-2-yl)benzoic acid (0.61 g, 3.0 mmol) as raw materials.
MS (ESI pos.) m/z: 393 [M+H]+

### Reference Example 26: N-(2-Cyanoethyl)-5-methyl-2-(2H-1,2,3-triazol-2-yl)benzamide

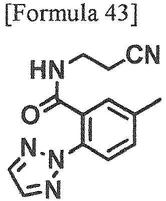

The titled compound (0.61 g, colorless solid) was prepared in the same manner as in Reference Example 19 using 3-aminopropanenitrile (0.20 mL, 2.7 mmol) and 5-methyl-2-(2H-1,2,3-triazol-2-yl)benzoic acid (0.61 g, 3.0 mmol) as raw materials.
MS (ESI pos.) m/z: 256 [M+H]+

### Reference Example 27: N-{2-[5-(5-Fluoropyridin-2-yl)-1,2,4-oxadiazol-3-yl]ethyl}-5-methyl-2-(2H-1,2,3-triazol-2-yl)benzamide

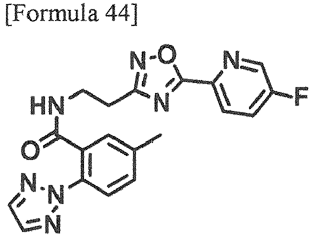

An aqueous 50% hydroxylamine solution (0.057 mL, 0.86 mmol) was added to a solution of N-(2-cyanoethyl)-5-methyl-2-(2H-1,2,3-triazol-2-yl)benzamide (0.20 g, 0.78 mmol) prepared in Reference Example 26 in EtOH (0.8 mL), followed by stirring at 90°C for 1 hour. After cooling to room temperature, an aqueous 50% hydroxylamine solution (0.057 mL, 0.86 mmol) was further added thereto, and the mixture was stirred again at 90°C for 1 hour. After cooling at room temperature, the reaction solution was concentrated under reduced pressure. A solution of the resulting residue in DMF (0.3 mL) was added to a solution of 5-fluoropyridin-2-carboxylic acid (0.12 g, 0.82 mmol) and carbonyldiimidazole (0.15 g, 0.94 mmol) in DMF (0.5 mL) that was stirred in advance at 40°C for 1 hour. The reaction solution was warmed to 90°C, followed by stirring for 15 hours. Water was added to the reaction mixture, followed by extraction with EtOAc. The organic layer was washed with water and brine and was dried with Na₂SO₄. The desiccant was then removed by filtration, and the solvent was distilled off under reduced pressure. The resulting residue was purified by column chromatography (KP-NH: 28 g, hexane/EtOAc = 75/25 to 0/100) to give the titled compound (0.040 g, colorless and amorphous).
MS (ESI pos.) m/z: 394 [M+H]+

### Reference Example 28: N-[2-(1H-Imidazol-4-yl)ethyl]-5-methyl-2-(2H-1,2,3-triazol-2-yl)benzamide

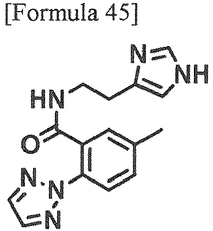

DIPEA (1.4 mL, 8.2 mmol) and HATU (0.74 g, 2.0 mmol) were added to a solution of 2-(1H-imidazol-4-yl)ethanamine dihydrochloride (0.30 g, 1.6 mmol) and 5-methyl-2-(2H-1,2,3-triazol-2-yl)benzoic acid (0.33 g, 1.6 mmol) in DMF (5 mL), followed by stirring at room temperature for 15 hours. Water was added to the reaction mixture, followed by extraction with EtOAc. The organic layer was washed with water and brine, and the solvent was distilled off under reduced pressure. The resulting residue was purified by column chromatography (KP-NH: 28 g, CHCl₃/MeOH = 100/0 to 80/20) to give the titled compound (0.18 g, colorless and amorphous).
MS (ESI pos.) m/z: 297 [M+H]+

### Reference Example 29: N-{2-[1-(5-Fluoropyridin-2-yl)-1H-imidazol-4-yl]ethyl]-5-methyl-2-(2H-1,2,3-triazol-2-yl)benzamide

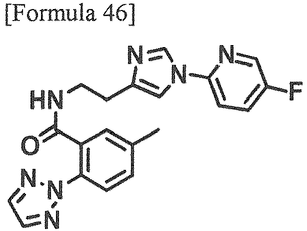

The titled compound (0.13 g, brown and amorphous) was prepared in the same manner as in Reference Example 3 using N-[2-(1H-imidazol-4-yl)ethyl]-5-methyl-2-(2H-1,2,3-triazol-2-yl)benzamide (0.18 g, 0.60 mmol) prepared in Reference Example 28 and 2-bromo-5-fluoropyridine (0.13 g, 0.72 mmol) as raw materials.
MS (ESI pos.) m/z: 392 [M+H]+

### Reference Example 30: Tert-Butyl methyl{2-[1-(5-fluoropyndin-2-yl)-1H-pyrazol-3-yl]ethyl carbamate

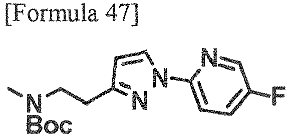

The titled compound (0.31 g, colorless oil) was prepared in the same manner as in Reference Example 17 using tert-butyl{2-[1-(5-fluoropyridin-2-yl)-1H-pyrazol-3-yl]ethyl}carbamate (0.3 g, 0.98 mmol) prepared in Reference Example 16 as a raw material and using MeI (0.073 mL, 1.18 mmol).
MS (ESI pos.) m/z: 321 [M+H]+

### Reference Example 31: 2-[1-(5-Pluoropyridin-2-yl)-1H-pyrazol-3-yl]-N-methylethanamine hydrochloride

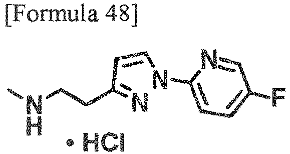

The titled compound (0.2 g, colorless solid) was prepared in the same manner as in Reference Example 18 using tert-butyl methyl{2-[1-(5-fluoropyridin-2-yl)-1H-pyrazol-3-yl]ethyl}carbamate (0.31 g, 0.98 mmol) prepared in Reference Example 30 as a raw material. MS (ESI pos.) m/z: 221 [M+H]+

Reference Example 32: 2-[1-(5-Fluoropyridin-2-yl)-1H-pyrazol-3-yl]ethanamine dihydrochloride

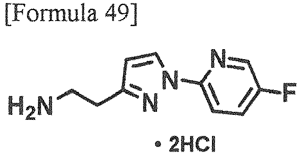

The titled compound (0.34 g, yellowish brown solid) was prepared in the same manner as in Reference Example 18 using tert-butyl{2-[1-(5-fluoropyridin-2-yl)-1H-pyrazol-3-yl]ethyl}carbamate (0.46 g, 1.49 mmol) prepared in Reference Example 16 as a raw material. MS (ESI pos.) m/z: 207 [M+H]+

### Reference Example 33: 4-Fluoro-N-{2-[1-(5-fluoropyridin-2-yl)-1H-pyrazol-3-yl]ethyl}-2-(2H-1,2,3-triazol-2-yl)benzamide

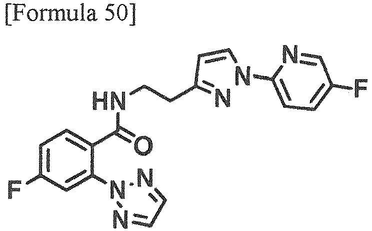

HOBt·H₂O (39 mg, 0.25 mmol) and EDC·HCl (0.049 g, 0.25 mmol) were added to a solution of the free form of 2-[1-(5-fluoropyridin-2-yl)-1H-pyrazol-3-yl]ethanamine dihydrochloride (0.035 g, 0.17 mmol) prepared in Reference Example 32 and 4-fluoro-2-(2H-1,2,3-triazol-2-yl)benzoic acid (0.039 g, 0.19 mmol) in DMF (1.5 mL) at room temperature, followed by stirring overnight. An aqueous NaHCO₃ solution was added to the reaction solution, followed by extraction with EtOAc. The organic layer was washed with water and brine and was dried with Na₂SO₄. The desiccant was removed by filtration, and the solvent was then distilled under reduced pressure. The resulting residue was purified by column chromatography (HP-Sil: 10 g, hexane/EtOAc = 88/12 to 0/100) to give the titled compound (0.048 g, colorless oil).
MS (ESI pos.) m/z: 396 [M+H]+

Compounds of Reference Examples 34 to 37 were prepared in the same manner as in Reference Example 33 The structural formulas, the names, and the MS data of the resulting compounds are shown in Table 1

**[Table 1]**

| Reference Example No | Structural Formula | Name of Compound | MS (ESI pos.) m/z |
|---|---|---|---|
| Reference Example 34 | 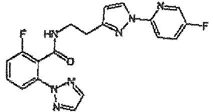 | 2-fluoro-N-{2[1-(5-fluoropyridin-2-yl)-1H-pyrazol-3-yl] ethyl}-6-(2H-1,2,3-triazol-2-yl)benzamide | 396(M+H)+ |
| Reference Example 35 | 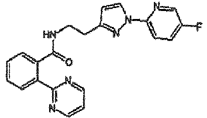 | N-{2-[1-(5-fluoropyridin-2-yl)-1H-pyrazol-3-yl]ethyl}-2-(pyrimidin-2-yl)benzamide | 389(M+H)+ |
| Reference Example 36 | 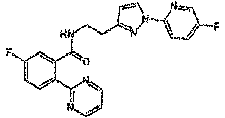 | 5-fluoro-N-{2-[1-(6-fluoropyridin-2-yl)-1H-pyrazol-3-yl] ethyl}-2-(pyridin-2-yl)benzamide | 407(M+H)+ |
| Reference Example 37 | 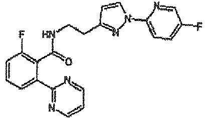 | 2 fluoro N-{2-[1-(5-fluoropyridin-2-yl)-1H-pyrazol-3-yl] ethyl}-6-(pyrimidin-2-yl)benzamide | 407(M+H)+ |

### Reference Example 38 Ethyl (2E)-3-[1-(Tetrahydro-2H-pyran-2-yl)-1H-pyrazol-3-yl]prop-2-enoate

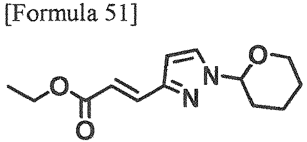

The titled compound (5.15 g, colorless oil) was prepared in the same manner as in Reference Example 14 using 2-(tetrahydro-pyran-2-yl)-2H-pyrazol-3-carbaldehyde (3 96 g, 22 0 mmol) as a raw material
MS (ESI pos.) m/z 251 [M+H]+

### Reference Example 39: 3-[1-(Tetrahydro-2H-pyran-2-yl)-1H-pyrazol-3-yl]propanoic acid

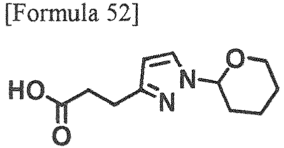

The titled compound (2.85 g, colorless oil) was prepared in the same manner as in Reference Example 15 using ethyl (2E)-3-[1-(tetrahydro-2H-pyran-2-yl)-1H-pyrazol-3-yl]prop-2-enoate (3.37 g, 13.4 mmol) prepared in Reference Example 38 as a raw material.
MS (ESI pos.) m/z: 247 [M+Na]+

### Reference Example 40: Benzyl{2-[1-(tetrahydro-2H-pyran-2-yl)-1H-pyrazol-3-yl]ethyl} carbamate

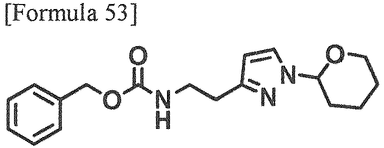

The titled compound (0.60 g, light yellow oil) was prepared in the same manner as in Reference Example 16 using 3-[1-(tetrahydro-2H-pyran-2-yl)-1H-pyrazol-3-yl]propanoic acid (2.45 g, 10.9 mmol) prepared in Reference Example 39 and benzyl alcohol (1.36 mL, 13.1 mmol) as raw materials.
MS (ESI pos.) m/z: 352 [M+Na]+

### Reference Example 41: 2-[1-(Tetrahydro-2H-pyran-2-yl)-1H-pyrazol-3-yl]ethanamine

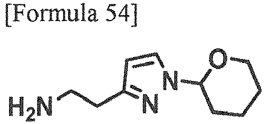

Pd/C 10% (55% water) (0.035 g, 0.18 mmol) was added to a solution of benzyl{2-[1-(tetrahydro-2H-pyran-2-yl)-1H-pyrazol-3-yl]ethyl}carbamate (0.60 g, 1.81 mmol) prepared in Reference Example 40 in EtOH (12 mL). After hydrogen substitution, the mixture was stirred at room temperature for 1 hour. Insoluble matter was removed by filtration through Celite (registered trademark), and then the solvent was distilled off under reduced pressure to give the titled compound (0.41 g, colorless oil).
MS (ESI pos.) m/z: 196 [M+H]+

### Reference Example 42: N-{2-[1-(Tetrahydro-2H-pyran-2-yl)-1H-pyrazol-3-yl]ethyl}-2-(2H-1,2,3-triazol-2-yl)benzamide

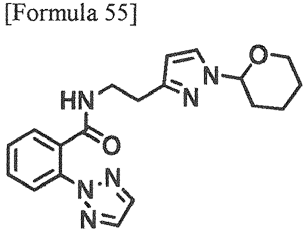

The titled compound (0.47 g, 1.29 mmol, yellow oil) was prepared in the same manner as in Reference Example 1 using 2-[1-(tetrahydro-2H-pyran-2-yl)-1H-pyrazol-3-yl]ethanamine (0.35 g, 1.81 mmol) prepared in Reference Example 41 and 2-(2H-1,2,3-triazol-2-yl)benzoic acid (0.34 g, 1.81 mmol) as raw materials.
MS (ESI pos.) m/z: 367 [M+H]+

Compounds of Reference Examples 43 and 44 were prepared in the same manner as in Reference Example 42. The structural formulas, the names, and the MS data of the resulting compounds are shown in Table 2.

**[Table 2]**

| Reference Example No. | Structural Formula | Name of Compound | MS (ESI pos.) m/z |
|---|---|---|---|
| Reference Example 43 | 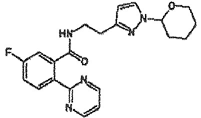 | 5-fluoro-2-(pyrimidin-2-yl)-N-[2-[1-(tetrahydro-2H-pyran-2-yl)-1H-pyrazol-3-yl]ethyl}benzamide | 396(M+H)+ |
| Reference Example 44 | 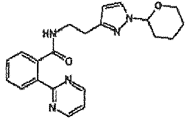 | 2-(pyrimidin-2-yl)-N-{2-[1-(tetrahydro-2H-pyran-2-yl)-1H-pyrazol-3-yl]ethyl}benzamide | 378(M+H)+ |

### Reference Example 45: N-Ethyl-N-{2-[1-(tetrahydro-2H-pyran-2-yl)-1H-pyrazol-3-yl]ethyl}-2-(2H-1,2,3-triazol-2-yl)benzamide

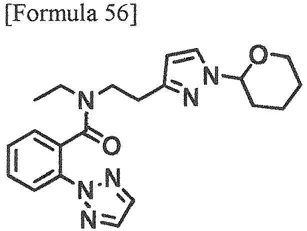

The titled compound (0.37 g, yellow oil) was prepared in the same manner as in Reference Example 17 using N-{2-[1-(tetrahydro-2H-pyran-2-yl)-1H-pyrazol-3-yl]ethyl}-2-(2H-1,2,3-triazol-2-yl)benzamide (0.47 g, 1.29 mmol) prepared in Reference Example 42 and ethyl iodide (0.13 mL, 1.55 mmol) as raw materials.
MS (ESI pos.) m/z: 395 [M+H]+

Compounds of Reference Examples 46 and 47 were prepared in the same manner as in Reference Example 45. The structural formulas, the names, and the MS data of the resulting compounds are shown in Table 3.

**[Table 3]**

| Reference Example No. | Structural Formula | Name of Compound | MS (ESI pos.) m/z |
|---|---|---|---|
| Reference Example 46 | 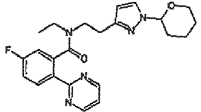 | N-ethyl-5-fluoro-2-(pyrimidin-2-yl)-N-{2-[1-(tetrahydro -2H-pyran-2-yl)-1H-pyrazol-3-yl]ethyl}benzamide | 424(M+H)+ |
| Reference Example 47 | 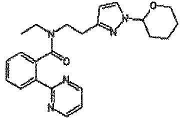 | N-ethyl-2-(pyrimidin-2-yl)-N-[2-[1-(tetrahydin-2H-pyran-2-yl)-1H-pyrazol-3-yl]ethyl}benzamide | 406(M+H)+ |

### Reference Example 48: N-Ethyl-N-[2-(1H-pyrazol-3-yl)ethyl]-2-(2H-1,2,3-triazol-2-yl)benzamide

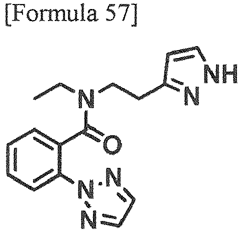

A solution of 4 mol/L HCl-1,4-dioxane (0.11 mL, 0.47 mmol) was added to a solution of N-ethyl-N-{2-[1-(tetrahydro-2H-pyran-2-yl)-1H-pyrazol-3-yl]ethyl}-2-(2H-1,2,3-triazol-2-yl)benzamide (0.37 g, 0.93 mmol) prepared in Reference Example 45 in EtOH (10 mL) at room temperature, followed by stirring overnight. A solution of 4 mol/L HCl-1,4-dioxane (0.23 mL, 0.93 mmol) was further added thereto, followed by stirring at room temperature for 1.5 hours. Furthermore, a solution of 4 mol/L HCl-1,4-dioxane (0.23 mL, 0.93 mmol) was added thereto, followed by stirring in an oil bath (50°C) for 3 hours. A saturated aqueous NaHCO₃ solution was added to the reaction mixture, followed by extraction with CHCl₃. The solvent was distilled off under reduced pressure. The resulting residue was then purified by column chromatography (SNAP KP-NH: 28 g, hexane/EtOAc = 75/25 to 0/100) to give the titled compound (0.24 g, yellow oil).
MS (ESI pos.) m/z: 311 [M+H]+

Compounds of Reference Examples 49 and 50 were prepared in the same manner as in Reference Example 48. The structural formulas, the names, and the MS data of the resulting compounds are shown in Table 4.

**[Table 4]**

| Reference Example No. | Structural Formula | Name of Compound | MS (ESI pos.) m/z |
|---|---|---|---|
| Reference Example 49 | 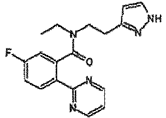 | N-ethyl-5-fluoro-N-[2-(1H-pyrazol-yl)ethyl]-2-(pyrimidin-2-yl)benzamide | 340(M+H)+ |
| Reference Example 50 | 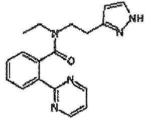 | N-ethyl-N-(2-(1H-pyrazol-3-yl)ethyl]-2-(pyrimidin-2-yl) benzamide | 322(M+H)+ |

### Reference Example 51: N-(2-Cyanoethyl)-2-(2H-1,2,3-triazol-2-yl)benzamide

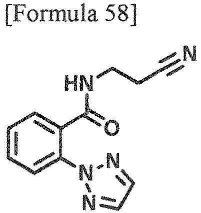

The titled compound (0.56 g, colorless solid) was prepared in the same manner as in Reference Example 19 using 3-aminopropanenitrile (0.22 mL, 2.95 mmol) and 2-(2H-1,2,3-triazol-2-yl)benzoic acid (0.60 g, 2.95 mmol) as raw materials.
MS (ESI pos.) m/z: 242 [M+H]+

### Reference Example 52: N-{2-[5-(5-Fluoropyridin-2-yl)-1,2,4-oxadiazol-3-yl]ethyl)-2-(2H-1,2,3-triazol-2-yl)benzamide

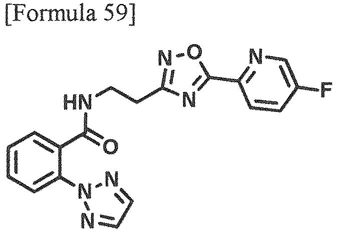

The titled compound (0.22 g, light yellow oil) was prepared in the same manner as in Reference Example 27 using N-(2-cyanoethyl)-2-(2H-1,2,3-triazol-2-yl)benzamide (0.56 g, 2.21 mmol) prepared in Reference Example 51 as a raw material.
MS (ESI pos.) m/z: 380 [M+H]+

Compounds of Reference Examples 53 to 56 were prepared in the same manner as in Reference Example 52. The structural formulas, the names, and the MS data of the resulting compounds are shown in Table 5.

**[Table 5]**

| Reference Example No. | Structural Formula | Name of Compound | MS (ESI pos.) mₕ |
|---|---|---|---|
| Reference Example 53 | 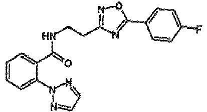 | N-{2-[5-(9-fluoropherryl)-1,2,4-oxadiazol-3-yl]ethyl}-2-(2H-1,2,3-triazol-2-yl)benzamide | 379(M+H)+ |
| Reference Example 54 | 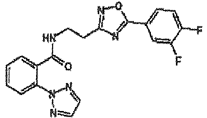 | N-{2-[5-(3,4-drifluorophenyl)-1,2,4-oxadiazol-3-yl] ethyl}-2-(2H-1,2,3-triazol-2-yl)benzamide | 397(M+H)+ |
| Reference Example 55 | 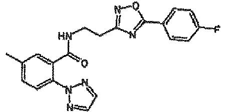 | N-{2-[5-(4-fluorophenyl)-1,2,4-oxadiazol-3-yl]ethyl}-5-methyl-2-(2H-1,2,3-triazol-2-yl)benzamide | 393(M+H)+ |
| Reference Example 56 | 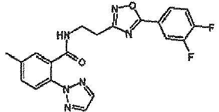 | N-{2-[5-(3,4-difluorophenyl)-1,2,4-oxadiazol-3-yl] ethyl}-5-methyl-2-(2H-1,2,3-triazol-2-yl)benzamide | 411(M+H)+ |

Reference Example 57: Tert-Butyl(2-cyanoethyl)ethylcarbamate

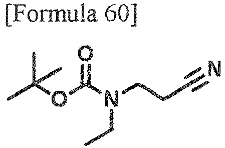

The titled compound (4.70 g, light yellow oil) was prepared in the same manner as in Reference Example 17 using tert-butyl(2-cyanoethyl)carbamate (7.29 g, 42.8 mmol) and ethyl iodide (5.1 mL, 64.2 mmol) as raw materials.
MS (ESI pos.) m/z: 199 [M+H]+

### Reference Example 58: Tert-Butyl ethyl{2-[5-(4-fluorophenyl)-1,2,4-oxadiazol-3-yl]ethyl}carbamate

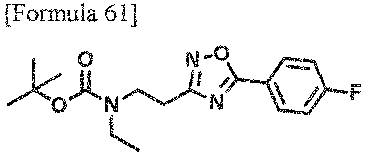

An aqueous 50% hydroxylamine solution (2.0 mL) was added to a solution of tert-butyl(2-cyanoethyl)ethylcarbamate (4.70 g, 23.7 mmol) prepared in Reference Example 57 in ethanol (20 mL), followed by stirring at 90°C for 5 hours. After cooling at room temperature, the solvent of the reaction solution was distilled under reduced pressure. A saturated aqueous sodium chloride solution was added to the resulting residue, followed by extraction with chloroform. After passing through a phase separator, the solvent was distilled off under reduced pressure. The resulting residue was washed with diethyl ether, then collected by filtration, and dried while heating under reduced pressure to give tert-butyl[3-amino-3-(hydroxyimino)propyl]ethylcarbamate (4.60 g, 19.9 mmol). The titled compound (1.45 g, colorless oil) was prepared in the same manner as in Reference Example 27 using the resulting tert-butyl[3-amino-3-(hydroxyimino)propyl]ethylcarbamate (1.50 g, 6.49 mmol) as a raw material.
MS (ESI pos.) m/z: 336 [M+H]+

### Reference Example 59: Tert-Butyl {2-[5-(3,4-difluorophenyl)-1,2,4-oxadiazol-3-yl]ethyl}ethylcarbamate

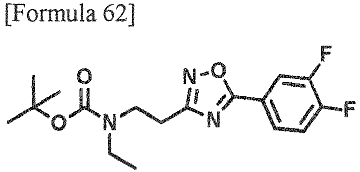

3,4-Benzonitrile (0.90 g, 6.49 mmol), zinc chloride (0.44 g, 3.24 mmol), and p-toluenesulfonic acid monohydrate (0.62 g, 3.24 mmol) were added to a solution of tert-butyl[3-amino-3-(hydroxyimino)propyl]ethylcarbamate (1.50 g, 6.49 mmol) prepared in Reference Example 58 in DMF (30 mL), followed by stirring at 90°C for 2 hours. After cooling at room temperature, water was added to the reaction solution, followed by extraction with ethyl acetate. The extracted organic layer was washed with a saturated aqueous sodium bicarbonate solution and a saturated aqueous sodium chloride solution in this order and was dried with anhydrous magnesium sulfate. The desiccant was then removed by filtration, and the solvent was distilled off under reduced pressure. The resulting residue was purified by column chromatography (HP-Sil: 10 g, hexane/EtOAc = 80/20 to 20/80) to give the titled compound (0.22 g, light yellow oil)
MS (ESI pos.) m/z: 376 [M+Na]+

A compound of Reference Example 60 was prepared in the same manner as in Reference Example 59. The structural formula, the name, and the MS data of the resulting compound are shown in Table 6.

**[Table 6]**

| Reference Example No. | Structural Formula | Name of Compound | MS (ESI pos.) m/z |
|---|---|---|---|
| Reference Example 60 | 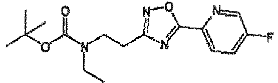 | tert-butylethyl{2-[5-(5-fluoropyridin-2-y))-1,2.4-oxadiazol-3-yl]ethyl}carbamate | 359(M+Na)+ |

### Reference Example 61: 2-[5-(3,4-Difluorophenyl)-1,2,4-oxadiazol-3-yl)-N-ethylethanamine

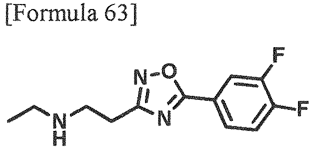

Trifluoroacetic acid (1 mL) was added to a solution of tert-butyl {2-[5-(3,4-difluorophenyl)-1,2,4-oxadiazol-3-yl]ethyl}ethylcarbamate (0.22 g, 0.62 mmol) prepared in Reference Example 59 in chloroform (5 mL), followed by stirring at room temperature for 4 hours. A saturated aqueous sodium bicarbonate solution was added to the reaction solution, followed by extraction with chloroform. The extracted organic layer was washed with a saturated aqueous sodium chloride solution and was then allowed to pass through a phase separator. The solvent was distilled offunder reduced pressure to give the titled compound (0.10 g, 0.40 mmol, light yellow oil).
MS (ESI pos.) m/z: 254 [M+H]+

Compounds of Reference Examples 62 and 63 were prepared in the same manner as in Reference Example 61. The structural formulas, the names, and the MS data of the resulting compounds are shown in Table 7.

**[Table 7]**

| Reference Example No. | Structural Formula | Name of Compound | MS (ESI pos.) m/z |
|---|---|---|---|
| Reference Example 62 | 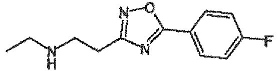 | N-ethyl-2-[5-(4-fluorophenyl)-1,2,4-oxadiazol-3-yl] ethaneamine | 236(M+H)+ |
| Reference Example 63 | 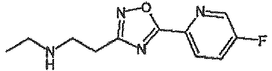 | N-ethyl-2-[5-(5-fluoropyridin-2-yl)-1,2,4-oxadiazol-3-yl]ethaneamine | 237(M+H)+ |

### Example 1: N-Ethyl-N-{2-[1-(5-fluoropyridin-2-yl)-1H-pyrazol-4-yl]ethyl}-5-methyl-2-(2H-1,2,3-triazol-2-yl)benzamide

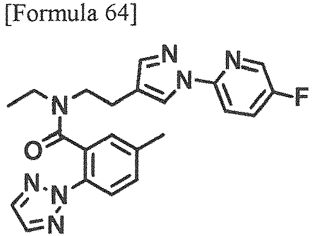

60% NaH (0.019 g, 0.44 mmol) was added to a solution of N-{2-[1-(5-fluoropyridin-2-yl)-1H-pyrazol-4-yl]ethyl}-5-methyl-2-(2H-1,2,3-triazol-2-yl)benzamide (0.14 g, 0.37 mmol) prepared in Reference Example 3 in DMF (1.2 mL), followed by stirring at room temperature for 1 hour. EtI (0.032 mL, 0.40 mmol) was dropwise added to the reaction solution, followed by stirring at room temperature for 15 hours. Water was added to the reaction solution, followed by extraction with EtOAc. The organic layer was washed with water and brine and was dried with Na₂SO₄. The desiccant was then removed by filtration, and the solvent was distilled off under reduced pressure. The resulting residue was purified by column chromatography (HP-Sil: 25 g, hexane/EtOAc = 80/20 to 0/100). The resulting solid was washed with Et₂O with stirring, followed by filtration to give the titled compound (0.11 g, colorless solid).
LCMS retention time: 5.70 min (Condition 1)
MS (ESI pos.) m/z: 420 [M+H]+

### Example 2: N-{2-[1-(5-Fluoropyridin-2-yl)-1H-pyrazol-4-yl]ethyl}-N,5-dimethyl-2-(2H-1,2,3-triazol-2-yl)benzamide

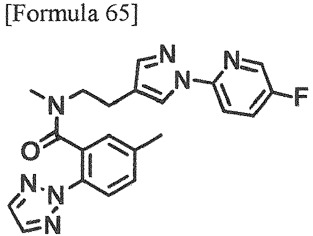

The titled compound (0.044 g, colorless solid) was prepared in the same manner as in Example 1 using N-{2-[1-(5-fluoropyridin-2-yl)-1H-pyrazol-4-yl]ethyl}-5-methyl-2-(2H-1,2,3-triazol-2-yl)benzamide (0,06 g, 0.15 mmol) prepared in Reference Example 3 and MeI (0.010 mL, 0.17 mmol) as raw materials.
LCMS retention time: 5.39 min (Condition 1)
MS (ESI pos.) m/z: 406 [M+H]+

### Example 3: N-Ethyl-N-{2-[1-(5-fluoropyridin-2-yl)-1H-pyrazol-4-yl]ethyl}-2-(2H-1,2,3-triazol-2-yl)benzamide

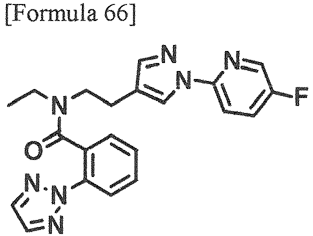

The titled compound (0.043 g, colorless solid) was prepared in the same manner as in Example 1 using N-{2-[1-(5-fluoropyridin-2-yl)-1H-pyrazol-4-yl]ethyl}-2-(2H-1,2,3-triazol-2-yl)benzamide (0.05 g, 0.13 mmol) prepared in Reference Example 4 and EtI (0.012 mL, 0.15 mmol) as raw materials.
LCMS retention time: 5.41 min (Condition 1)
MS (ESI pos.) m/z: 406 [M+H]+

### Example 4: N-Ethyl-N- {2-[1-(5-fluoropyridin-2-yl)-1H-pyrazol-3-yl]ethyl}-2-(2H-1,2,3-triazol-2-yl)benzamide

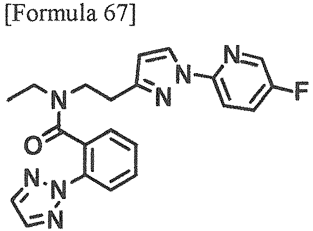

2-(2H-1,2,3-Triazol-2-yl)benzoic acid (0.02 g, 0.1 mmol), HOBt·H₂O (0.022 g, 0.14 mmol), and EDC·HCl (0.027 g, 0.14 mmol) were added to a solution of N-ethyl-2-[1-(5-fluoropyridin-2-yl)-1H-pyrazol-3-yl]ethanamine (0.022 g, 0.09 mmol) prepared in Reference Example 10 in DMF (0.8 mL), followed by stirring at room temperature for 2 hours. An aqueous NaHCO₃ solution was added to the reaction mixture, followed by extract with EtOAc. The solvent was distilled off under reduced pressure. The resulting residue was purified by column chromatography (HP-Sil: 10 g, hexane/EtOAc = 80/20 to 0/100, KP-NH: 11 g, hexane/EtOAc = 88/12 to 0/100), followed by washing with Et₂O with stirring to give the titled compound (0.013 g, colorless solid).
LCMS retention time: 5.54 min (Condition 1)
MS (ESI pos.) m/z: 406 [M+H]+

### Example 5: N-{2-[1-(5-Fluoropyridin-2-yl)-1H-pyrazol-3-yl]ethyl}-N,5-dimethyl-2-(2H-1,2,3-triazol-2-yl)benzamide

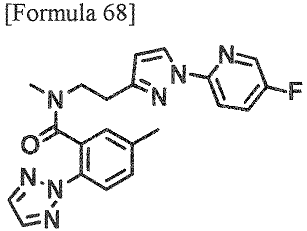

The titled compound (0.014 g, yellow oil) was prepared in the same manner as in Example 4 using N-methyl-2-[1-(5-fluoropyridin-2-yl)-1H-pyrazol-3-yl]ethanamine (0.062 g, 0.28 mmol) prepared in Reference Example 11 and 5-methyl-2-(2H-1,2,3-triazol-2-yl)benzoic acid (0.11 g, 0.56 mmol) as raw materials.
LCMS retention time: 5.50 min (Condition 1)
MS (ESI pos.) m/z: 406 [M+H]+

### Example 6: N-Ethyl-N-{2-[1-(5-fluoropyridin-2-yl)-1H-pyrazol-3-yl]ethyl}-5-methyl-2-(pyrimidin-2-yl)benzamide

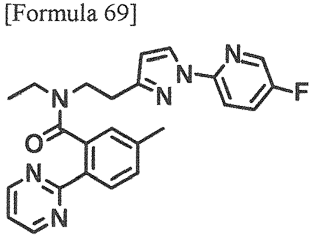

The titled compound (0.061 g, light orange solid) was prepared in the same manner as in Example 4 using N-ethyl-2-[1-(5-fluoropyridin-2-yl)-1H-pyrazol-3-yl]ethanamine (0.063 g, 0.27 mmol) prepared in Reference Example 10 and 5-methyl-2-(pyrimidin-2-yl)benzoic acid (0.371 g, 1.96 mmol) as raw materials.
LCMS retention time: 1.83 min (Condition 2)
MS (ESI/APCI pos.) m/z: 431 [M+H]+

### Example 7: N-Ethyl-N-{2-[1-(5-fluoropyridin-2-yl)-1H-pyrazol-3-yl]ethyl]-5-fluoro-2-(2H-1,2,3-triazol-2-yl)benzamide

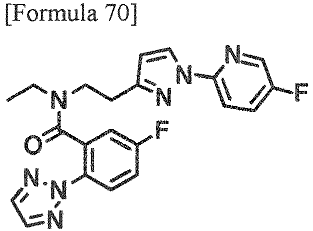

Et₃N (0.017 mL, 0.12 mmol), 5-fluoro-2-(2H-1,2,3-triazol-2-yl)benzoic acid (0.032 g, 0.15 mmol), HOBt·H₂O (0.019 g, 0.12 mmol), and EDC·HCl (0.024 g, 0.12 mmol) were added to a solution of N-ethyl-2-[1-(5-fluoropyridin-2-yl)-1H-pyrazol-3-yl]ethanamine hydrochloride (0.028 g, 0.10 mmol) prepared in Reference Example 18 in CHCl₃ (1.0 mL), followed by stirring at room temperature for 14 hours. Et₃N (0.017 mL, 0.12 mmol), 5-fluoro-2-(2H-1,2,3-triazol-2-yl)benzoic acid (0.032 g, 0.15 mmol), HOBt·H₂O (0.019 g, 0.12 mmol), and EDC·HCl (0.024 g, 0.12 mmol) were added to the reaction mixture, followed by stirring at room temperature for 1 hour and at 60°C for 3 hours. An aqueous NaHCO₃ solution (1.0 mL), water (1.0 mL), and CHCl₃ were added to the reaction mixture, followed by separation into an organic layer and an aqueous layer. The aqueous layer was extracted with CHCl₃ twice. The organic layers were combined and allowed to pass through ISOLUTE Phase Separator. The filtrate was then concentrated under reduced pressure. The resulting residue was purified by column chromatography (KP-NH: 11 g, hexane/EtOAc = 100/0 to 75/25) to give the titled compound (0.033 g, colorless solid).
LCMS retention time: 1.83 min (Condition 2)
MS (ESI/APCI pos.) m/z: 424 [M+H]+

### Example 8: N-Ethyl-N-{2-[1-(5-fluoropyridin-2-yl)-1H-pyrazol-3-yl]ethyl}-5-methyl-2-(2H-1,2,3-triazol-2-yl)benzamide

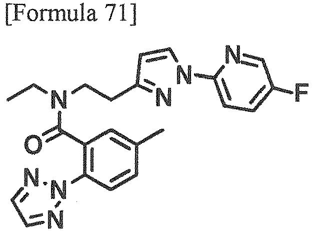

The titled compound (0.030 g, colorless solid) was prepared in the same manner as in Example 7 using N-ethyl-2-[1-(5-fluoropyridin-2-yl)-1H-pyrazol-3-yl]ethanamine hydrochloride (0.028 g, 0.10 mmol) prepared in Reference Example 18 and 5-methyl-2-(2H-1,2,3-triazol-2-yl)benzoic acid (0.031 g, 0.15 mmol) as raw materials.
LCMS retention time: 1.88 min (Condition 2)
MS (ESI/APCI pos.) m/z: 420 [M+H]+

### Example 9: N-{2-[5-(5-Fluoropyridin-2-yl)-1,2-oxazol-3-yl]ethyl}-N,5-dimethyl-2-(2H-1,2,3-triazol-2-yl)benzamide

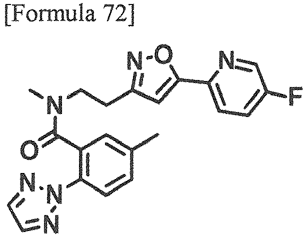

The titled compound (0.039 g, colorless solid) was prepared in the same manner as in Example 1 using N-{2-[5-(5-fluoropyridin-2-yl)-1,2-oxazol-3-yl]ethyl}-5-methyl-2-(2H-1,2,3-triazol-2-yl)benzamide (0.080 g, 0.20 mmol) prepared in Reference Example 21 and MeI (0.014 mL, 0.22 mmol) as raw materials.
LCMS retention time: 5.11 min (Condition 1)
MS (ESI pos.) m/z: 407 [M+H]+

### Example 10: N-Ethyl-N-{2-[5-(5-fluoropyridin-2-yl)-1,2-oxazol-3-yl]ethyl)-5-methyl-2-(2H-1,2,3-triazol-2-yl)benzamide

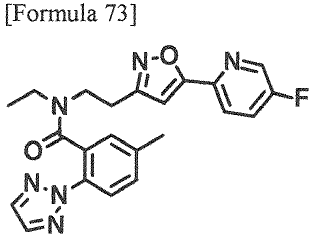

The titled compound (0.060 g, colorless solid) was prepared in the same manner as in Example 1 using N-{2-[5-(5-fluoropyridin-2-yl)-1,2-oxazol-3-yl]ethyl}-5-methyl-2-(2H-1,2,3-triazol-2-yl)benzamide (0.080 g, 0.20 mmol) prepared in Reference Example 21 and EtI (0.018 mL, 0.22 mmol) as raw materials.
LCMS retention time: 5.42 min (Condition 1)
MS (ESI pos.) m/z: 421 [M+H]+

### Example 11: N-Ethyl-N-{2-[3-(5-fluoropyridin-2-yl)-1,2-oxazol-5-yl]ethyl}-5-methyl-2-(2H-1,2,3-triazol-2-yl)benzamide

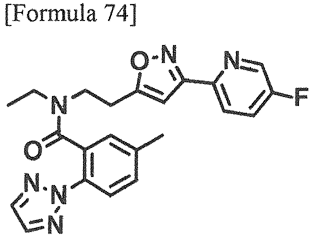

The titled compound (0.032 g, colorless solid) was prepared in the same manner as in Example 1 using N-{2-[3-(5-fluoropyridin-2-yl)-1,2-oxazol-5-yl]ethyl}-5-methyl-2-(2H-1,2,3-triazol-2-yl)benzamide (0.040 g, 0.10 mmol) prepared in Reference Example 25 and EtI (0.009 mL, 0.11 mmol) as raw materials.
LCMS retention time: 5.56 min (Condition 1)
MS (ESI pos.) m/z: 421 [M+H]+

### Example 12: N-Ethyl-N-{2-[5-(5-fluoropyridin-2-yl)-1,2,4-oxadiazol-3-yl]ethyl}-5-methyl-2-(2H-1,2,3-triazol-2-yl)benzamide

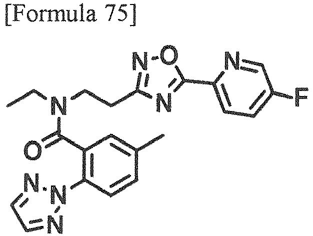

The titled compound (0.027 g, colorless solid) was prepared in the same manner as in Example 1 using N-{2-[5-(5-fluoropyridin-2-yl)-1,2,4-oxadiazol-3-yl]ethyl}-5-methyl-2-(2H-1,2,3-triazol-2-yl)benzamide (0.040 g, 0.10 mmol) prepared in Reference Example 27 and EtI (0.009 mL, 0.11 mmol) as raw materials.
LCMS retention time: 5.19 min (Condition 1)
MS (ESI pos.) m/z: 422 [M+H]+

### Example 13: N-Ethyl-N-{2-[1-(5-fluoropyridin-2-yl)-1H-imidazol-4-yl]ethyl}-5-methyl-2-(2H-1,2,3-triazol-2-yl)benzamide

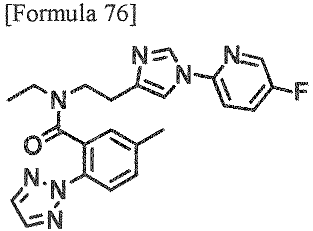

The titled compound (0.12 g, colorless and amorphous) was prepared in the same manner as in Example 1 using N-[2-[1-(5-fluoropyridin-2-yl)-1H-imidazol-4-yl]ethyl]-5-methyl-2-(2H-1,2,3-triazol-2-yl)benzamide (0.13 g, 0.32 mmol) prepared in Reference Example 29 and EtI (0.028 mL, 0.35 mmol) as raw materials.
LCMS retention time: 3.56 min (Condition 1)
MS (ESI pos.) m/z: 420 [M+H]+

### Example 14: N-{2-[1-(5-Fluoropyridin-2-yl)-1H-pyrazol-3-yl]ethyl}-N-methyl-2-(2H-1,2,3-triazol-2-yl)benzamide

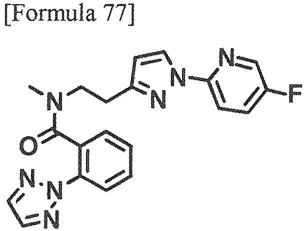

The titled compound (0.075 g, colorless and amorphous) was prepared in the same manner as in Example 7 using 2-[1-(5-fluoropyridin-2-yl)-1H-pyrazol-3-yl]-N-methylethanamine hydrochloride (0.080 g, 0.31 mmol) prepared in Reference Example 31 and 2-(2H-1,2,3-triazol-2-yl)benzoic acid (0.065 g, 0.34 mmol) as raw materials.
LCMS retention time: 1.74 min (Condition 2)
MS (ESI pos.) m/z: 392 [M+H]+

### Example 15: 5-Fluoro-N-{2-[1-(5-fluoropyridin-2-yl)-1H-pyrazol-3-yl]ethyl}-N-methyl-2-(2H-1,2,3-triazol-2-yl)benzamide

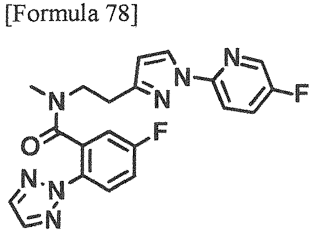

The titled compound (0.055 g, colorless and amorphous) was prepared in the same manner as in Example 7 using 2-[1-(5-fluoropyridin-2-yl)-1H-pyrazol-3-yl]-N-methylethanamine hydrochloride (0.050 g, 0.19 mmol) prepared in Reference Example 31 and 5-fluoro-2-(2H-1,2,3-triazol-2-yl)benzoic acid (0.044 g, 0.21 mmol) as raw materials.
LCMS retention time: 1.79 min (Condition 2)
MS (ESI pos.) m/z: 410 [M+H]+

### Example 16: 5-Chloro-N-{2-[1-(5-fluoropyridin-2-yl)-1H-pyrazol-3-yl]ethyl}-N-methyl-2-(2H-1,2,3-triazol-2-yl)benzamide

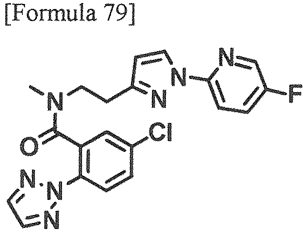

The titled compound (0.070 g, colorless and amorphous) was prepared in the same manner as in Example 7 using 2-[1-(5-fluoropyridin-2-yl)-1H-pyrazol-3-yl]-N-methylethanamine hydrochloride (0.050 g, 0.19 mmol) prepared in Reference Example 31 and 5-chloro-2-(2H-1,2,3-triazol-2-yl)benzoic acid (0.048 g, 0.21 mmol) as raw materials.
LCMS retention time: 1.82 min (Condition 2)
MS (ESI pos.) m/z: 426 [M+H]+

### Example 17: N-Ethyl-4-fluoro-N-{2-[1-(5-fluoropyridin-2-yl)-1H-pyrazol-3-yl]ethyl}-2-(2H-1,2,3-triazol-2-yl)benzamide

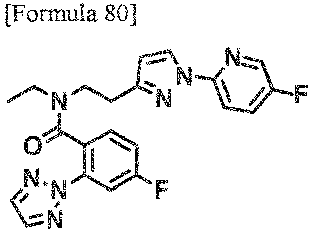

60% NaH (0.006 g, 0.15 mmol) was added to a solution of 4-fluoro-N-{2-[1-(5-fluoropyridin-2-yl)-1H-pyrazol-3-yl]ethyl}-2-(2H-1,2,3-triazol-2-yl)benzamide (0.048 g, 0.12 mmol) prepared in Reference Example 33 in DMF (0.4 mL), followed by stirring at room temperature for 30 min, followed by addition of EtI (0.011 mL, 0.13 mmol) thereto. The mixture was stirred at room temperature for 1 hour. Water was added to the reaction mixture, followed by extraction with EtOAc. The extracted organic layer was washed with brine and was dried with Na₂SO₄. The desiccant was then removed by filtration, and the solvent was distilled offunder reduced pressure. The resulting residue was purified by column chromatography (KP-NH: 28 g, hexane/EtOAc = 88/12 to 0/100). The resulting solid was washed with Et₂O with stirring, followed by filtration to give the titled compound (0.040 g, colorless solid).
LCMS retention time: 1.04 min (Condition 3)
MS (ESI pos.) m/z: 424 [M+H]+

Compounds of Examples 18 to 21 were prepared in the same manner as in Example 17. The structural formulas, the names, and the LCMS data of the resulting compounds are shown in Table 8.

**[Table 8]**

| Example No. | Structural Formula | Name of Compound | MS (ESI pos.) m/z | Conditions for Measurement by LCMS Retention time (min) |
|---|---|---|---|---|
| Example 18 | 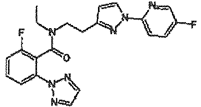 | N-ethyl-2-fluoro-N-{2-[1-(5-fluoropyridin-2-yl)-1H-pyrazol-3-yl]ethyl}-2-(2H-1,2,3-trfazol-2-yi) benzamide | 424(M+H)+ | Condition 3 1.01, 1.05 |
| Example 19 | 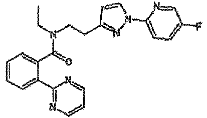 | N-ethyl-N-{2-[1-(5-fluoropyridin-2-yl)-1H-pyrazol-3-yl]ethyl}-2-(pyrimidin-2-yl) benzamide | 417(M+H)+ | Condition 3 0.98 |
| Example 20 | 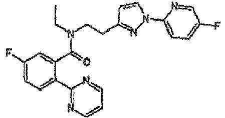 | N-ethyl-5-fluoro-N-{2[1-(5-fluoropyridin-2-yl)-1H-pyrazol-3-yl]ethyl}-2-(pyrimidin-2-yl) benzamide | 435(M+H)+ | Condition 3 1.02 |
| Example 21 | 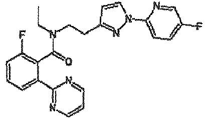 | N-ethyl-2-fluoro-N-{2-[1-(5-fluoropyridin-2-yl)-1H-pyrazol-3-yl]ethyl}-6-(pyrimidin-2-yl) benzamide | 435(M+H)⁺ | Condition 3 0.99, 1.04 |

### Example 22: N-Ethyl-N-{2-[1-(4-fluorophenyl)-1H-pyrazol-3-yl]ethyl}-2-(2H-1,2,3-triazol-2-yl)benzamide

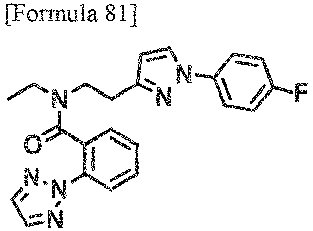

1-Bromo-4-fluorobenzene (0.042 g, 0.24 mmol), trans-N,N'-dimethylcyclohexane-1,2-diamine (0.026 mL, 0.16 mmol), copper iodide (0.008 g, 0.04 mmol), and cesium carbonate (0.11 g, 0.32 mmol) were added to a solution of N-ethyl-N-[2-(1H-pyrazol-3-yl)ethyl]-2-(2H-1,2,3-triazol-2-yl)benzamide (0.050 g, 0.16 mmol) prepared in Reference Example 48 in DMF (0.4 mL), followed by stirring at 90°C for 4 hours. 1-promo-4-fluorobenzene (0.042 g, 0.24 mmol) and trans-N,N'-dimethylcyclohexane-1,2-diamine (0.026 mL, 0.16 mmol) were further added thereto, followed by stirring at 90°C for 4 hours. The reaction mixture was purified by HPLC (Condition 2) and then further by PLC (NH silica: 0.75 mm, hexane/EtOAc = 1/1). The resulting oily material was freeze-dried to give the titled compound (0.028 g, colorless and amorphous).
LCMS retention time: 1.01 min (Condition 3)
MS (ESI pos.) m/z: 405 [M+H]+

Compounds of Examples 23 to 30 were prepared in the same manner as in Example 22. The structural formulas, the names, and the LCMS data of the resulting compounds are shown in Table 9.

**[Table 9]**

| Example No. | Structural Formula | Name of Compound | MS (ESI pos.) m/z | Conditions for Measurement by LCMS Retention time (min) |
|---|---|---|---|---|
| Example 23 | 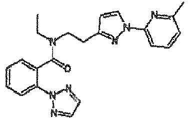 | N-ethyl-N-{2[1-(6-methylpyridin-2-yl)-1H-pyrazol-3-yl]ethyl}-2-(2H-1,2,3-triazol-2-yl) benzamide | 402(M+H)+ | Condition 3 1.03 |
| Example 24 | 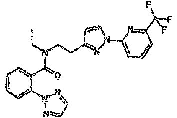 | N-ethyl-2-(2H-1,2,3-triazol-2-yl)-N-(2-{1-[6-(trifluoromethyl)pyridin-2-yl]-1H-pyrazol-3-yl} ethyl)benzamide | 456(M+H)+ | Condition 3 1.13 |
| Example 25 | 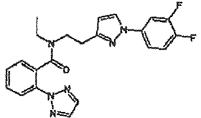 | N-{2-[1-(3,4-diftuorophenyl)-1H-pyrazol-3-yl] ethyl}-N-ethyl-2-(2H-1,2,3-triazol-2-yl) benzamide | 423(M+H)+ | Condition 3 1.06 |
| Example 26 | 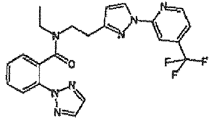 | N-ethyl-2-(2H-1,2,3-triazol-2-yl)-N-(2-{1-[4-(trifluoromethyl)pyridin-2-yl]-1H-pyrazol-3-yl} ethyl)benzamide | 456(M+H)+ | Condition 3 1.13 |
| Example 27 | 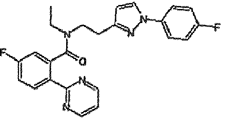 | N-ethyl-5-fluoro-N-{2-[1-(4-fluorophenyl)-9 H-pyrazol-3-yl]ethyl}-2-(pyrimidin-2-yl) benzamide | 434(M+H)+ | Condition 3 1.03 |
| Example 28 | 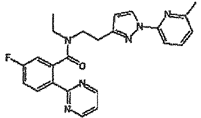 | N-ethyl-5-fluoro-N{2-(1-(6-methylpyridin-2-yl)-1H-pyrazol-3-yl]ethyl}-2-(pyrimidin-2-yl) benzamide | 431(M+H)+ | Condition 3 1.06 |
| Example 29 | 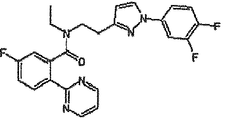 | N-{2-[1-(3,4-difluorophenyl)-1H-pyrazol-3-yl] ethyl}-N-ethyl-5-fluoro-2-(pyrimidin-2-yl) benzamide | 452(M+H)+ | Condition 3 1.08 |
| Example 30 | 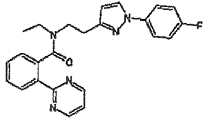 | N-ethyl-N-{2-[1-(4-fluorophenyl)-1H-pyrazol-3-yl]ethyl}-2-(pyrimidine-2-ynGenzamide | 416(M+H)+ | Condition 3 0.98 |

### Example 31: N-{2-[5-(4-Fluorophenyl)1,2,4-oxadiazol-3-yl]ethyl}-N-methyl-2-(2H-1,2,3-triazol-2-yl)benzamide

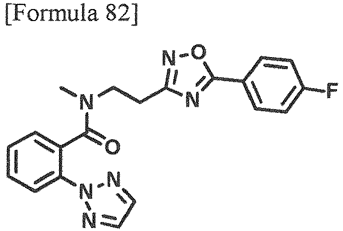

The titled compound (0.08 g, colorless and amorphous) was prepared in the same manner as in Example 1 using N-{2-[5-(4-fluorophenyl)-1,2,4-oxadiazol-3-yl]ethyl}-2-(2H-1,2,3-triazol-2-yl)benzamide (0.09 g, 0.24 mmol) prepared in Reference Example 53 and methyl iodide (0.18 mL, 0.29 mmol) as raw materials.
LCMS retention time: 0.95 min.(Condition 3)
MS (ESI pos.) m/z: 393 [M+H]+

Compounds of Examples 32 to 37 were prepared in the same manner as in Example 31. The structural formulas, the names, and the LCMS data of the resulting compounds are shown in Table 10.

**[Table 10]**

| Example No. | Structural Formula | Name of Compound | MS (ESI pos.) m/z | Conditions for Measurement by LCMS Retention time (min) |
|---|---|---|---|---|
| Example 32 | 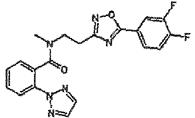 | N-{2-[5-(3,4-difluorophenyl)-1,2,4-oxadiazol-3-yl]ethyl}-N-methyl-2-(2H-1,2,3-triazol-2-yl) benzamide | 411(M+H)+ | Condition 3 0.99 |
| Example 33 | 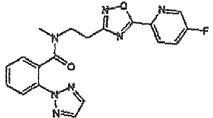 | N-{2-[5-(5-Huoropyridin-2-yl)-1,2,4-oxadiazol-3-yl]ethyl}-N-methyl-2-(2H-1,2,3-triazol-2-yl) benzamide | 394(M+H)+ | Condition 3 0.80 |
| Example 34 | 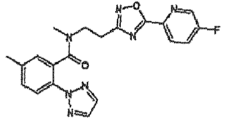 | N-{2-[5-(6-fluoropyridin-2-yl)-1,2,4-oxadiazol-3-yl]ethyl}-N,5-dimethyl-2-(2H-1,2,3-triazol-2-yl)benzamide | 448(M+H)+ | Condition 3 0.85 |
| Example 35 | 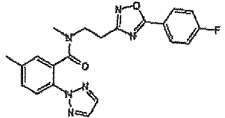 | N-{2-[5-{4-fluorophenyl)-1,2,4-oxadiazol-3-yl] ethyl]-N,5-dimethyl-2-(2H-1,2,3-triazol-2-yl) benzamide | 407(M+H)+ | Condition 3 1.02 |
| Example 36 | 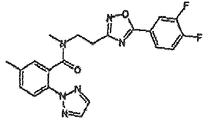 | N-{2-[5-(3,4-difluorophenyl)-1,2,4-oxadiazol-3-yl]ethyl}-N,5-dimethyl-2-(2H-1,2,3-triazol-3-yl)benzamide | 425(M+H)+ | Condition 3 1.06 |
| Example 37 | 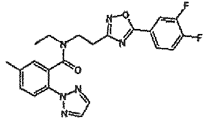 | N-ethyl-N-{2-[5-(3,4-difluorophenyl)-1,2,4-oxadiazol-3-yl]ethyl}-5-methyl-2-(2H-1,2,3-triazol-2-yl)benzamide | 439(M+H)+ | Condition 3 1.11 |

### Example 38: N-Ethyl-N-{2-[5-(4-fluorophenyl)-1,2,4-oxadiazol-3-yl]ethyl}-5-methyl-2-(2H-1,2,3-triazol-2-yl)benzamide

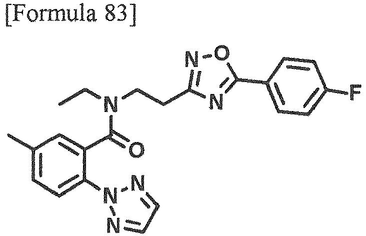

The titled compound (0.022 g, colorless solid) was prepared in the same manner as in Example 7 using 2-[5-(4-fluorophenyl)-1,2,4-oxadiazol-3-yl)-N-ethylethanamine (0.05 g, 0.21 mmol) prepared in Reference Example 62 and 5-methyl-2-(2H-1,2,3-triazol-2-yl)benzoic acid (0.043 g, 0.21 mmol) as raw materials.
LCMS retention time: 1.07 min.(Condition 3)
MS (ESI pos.) m/z: 421 [M+H]+

Compounds of Examples 39 to 46 were prepared in the same manner as in Example 38. The structural formulas, the names, and the LCMS data of the resulting compounds are shown in Table 11.

**[Table 11]**

| Example No | structural Formula | Name of Compound | MS (ESI pos.) m/z | Conditions for Measurement by LCMS Retention time (min) |
|---|---|---|---|---|
| Example 39 | 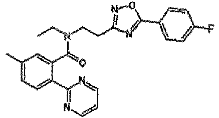 | N-ethyl-N-{2-[5-(4-fluorophenyl)-1,2,4-oxadiazol-3-yl]ethyl}-5-methyl-2-(pyrimidin-2 yl)benzamide | 432(M+H)+ | Condition 3 1.06 |
| Example 40 | 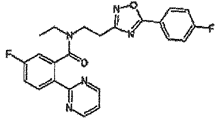 | N-ethyl-5-fluoro-N-{2-[5-(4-fluorophenyl) 1,2,4-oxadiazol-3-yl]ethyl}-2-(pyrimidin-2-yl) banzamide | 436(M+H)+ | Condition 3 1.04 |
| Example 41 | 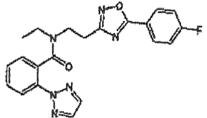 | N-ethyl-N-{2-[5-(4-fluorophenyl)-1,2,4-oxadiazol-3-yl]ethyl}-2-(2H-1,2,3-triazol-2-yl) benzamide | 407(M+H)+ | Condition 3 1.02 |
| Example 42 | 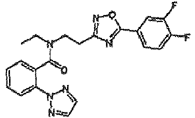 | N-ethyl-N-{2-[5-(3,4-difluorophenyl)-1,2,4-oxadiazol-3-yl]ethyl}-2-(2H 1 2 3-triazol-2-yl) benzamide | 425(M+H)+ | Condition 3 1.06 |
| Example 43 | 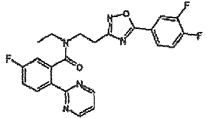 | N-{2-[5-(3,4-difluorophenyl)-1,2,4-oxadiazol-3-yl]ethyl}-N-ethyl-5-fluoro-2-(pyrimidin-2-yl) benzamide | 454(M+H)+ | Condition 3 1.08 |
| Example 44 | 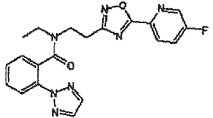 | N-ethyl-N-{2-[5-(5-fluoropyridin-2-yl)-1,2,4-oxidiazol-3-yl]ethyl}-2-(2H-1 2 3-triazol-2-yl) benzamide | 408(M+H)+ | Condition 3 0.87 |
| Example 45 | 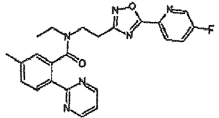 | N-ethyl-N-{2-[6-(5-fluoropyridin-2-yl)-1,2,4-oxadiazol-3-yl]-ethyl}-5-methyl-2-(pyrimidin-2-yl)benzamide | 433(M+H)+ | Condit on 3 0.89 |
| Example 46 | 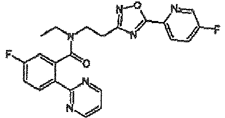 | N-ethyl-5-fluoro-N-{2-[5-(5-fluoropyridin-2-yl)-1,2,4-oxadiazol-3-yl]ethyl}-2-(pyrimidin-2-yl) benzamide | 437(M+H)+ | Condition 3 0.88 |

### Test Example (Measurement of orexin antagonist activity)

The antagonist activity of a test compound against the human orexin 1 receptor (hOX1R) or orexin 2 receptor (hOX2R) was measured by the method described in the document (Toshikatsu Okumura et al., Biochemical and Biophysical Research Communications, 280, 976-981, 2001) with appropriate modification. Chinese hamster ovary (CHO) cells forcibly expressing hOX1R or hOX2R were seeded into each well of a 96-well Black clear bottom plate (Nunc A/S) in the amount of 20000 cells per well and were cultured in a Ham's F-12 medium (Invitrogen, Inc.) containing 0.1 mM MEM non-essential amino acids (Invitrogen, Inc.), 0.5 mg/mL G418 (Invitrogen, Inc.), and 10% fetal calf serum (Invitrogen, Inc.) at 37°C in 5% CO₂ for 16 hours. The medium was removed, and then added to each well was 100 µL of an assay buffer (pH 7.4) containing 0.5 µM Fluo 4-AM ester (Dojindo Laboratories), wherein the buffer was composed of 25 mM HEPES (Dojindo Laboratories), a Hanks' balanced salt solution (Invitrogen, Inc.), 0.1% bovine serum albumin (Sigma-Aldrich Co., LLC.), 2.5 mM probenecid (Sigma-Aldrich Co., LLC.), and 200 µg/mL Amaranth (Sigma-Aldrich Co., LLC.), followed by incubation at 37°C in 5% CO₂ for 60 min. The assay buffer containing Fluo 4-AM ester was removed, and then 150 µL of an assay buffer solution containing 10 mM of the test compound dissolved in dimethyl sulfoxide was added to each well, followed by incubation for 30 min.

A peptide (Pyr-Pro-Leu-Pro-Asp-Ala-Cys-Arg-Gln-Lys-Thr-Ala-Ser-Cys-Arg-Leu-Tyr-Glu-Leu-Leu-His-Gly-Ala-Gly-Asn-His-Ala-Ala-Gly-Ile-Leu-Thr-Leu-NH₂: Peptide Institute, Inc.), which is a ligand having substitution of two amino acids in human orexin-A, was diluted with an assay buffer to a final concentration of 300 pM for hOX1R and to a final concentration of 3 nM for hOX2R. The reaction was started by adding 50 µL of this ligand solution to each well. The reaction was monitored by measuring the fluorescence of each well at each 1 second for 3 min with a Functional Drug Screening System (FDSS manufactured by Hamamatsu Photonics K.K.), and the antagonist activity was determined based on the maximum fluorescence value as an index of the intracellular Ca²⁺ concentration. The antagonist activity of a test compound was calculated by assuming that the fluorescence value of the well containing only a dilution buffer was 100% and that the fluorescence value of the well containing a buffer not containing the ligand or any test compound was 0%. The 50% inhibitory concentration (IC₅₀ value) of a test compound was determined from fluorescence values at various concentrations of the test compound.

Table 12 shows the IC₅₀ values of the inventive compounds.

**[Table 12]**

| Example No. | IC₅₀ value | | Example No. | IC₅₀ value | | Example No. | IC₅₀ value | |
|---|---|---|---|---|---|---|---|---|
| | OX1 (nM) | OX2 (nM) | | OX1 (nM) | OX2 (nM) | | OX1 (nM) | OX2 (nM) |
| 1 | 1.8 | 5.2 | 16 | 3.3 | 30.5 | 31 | 11.6 | 113.7 |
| 2 | 0.7 | 8.5 | 17 | 0.4 | 2.8 | 32 | 14.8 | 133.4 |
| 3 | 4.2 | 60.6 | 18 | 1.6 | 6.3 | 33 | 1193.7 | 2531.9 |
| 4 | 0.5 | 2.4 | 19 | 0.9 | 7.4 | 34 | 83.4 | 118.9 |
| 5 | 2.1 | 8.0 | 20 | 1.7 | 13.2 | 35 | 2.4 | 13.9 |
| 6 | 7.1 | 13.7 | 21 | 11.6 | 50.8 | 36 | 1.9 | 5.9 |
| 7 | 11.0 | 24.9 | 22 | 2.2 | 10.6 | 37 | 1.1 | 2.0 |
| 8 | 1.8 | 3.8 | 23 | 3.0 | 10.3 | 38 | 1.2 | 6.1 |
| 9 | 16.7 | 77.4 | 24 | 17.8 | 24.8 | 39 | 0.7 | 17.3 |
| 10 | 1.8 | 21.3 | 25 | 1.6 | 3.3 | 40 | 1.7 | 78.0 |
| 11 | 17.0 | 26.4 | 26 | 61.1 | 380.5 | 41 | 1.6 | 31.6 |
| 12 | 1.9 | 11.6 | 27 | 1.1 | 18.4 | 42 | 9.7 | 10.8 |
| 13 | 8.9 | 3.4 | 28 | 8.1 | 81.1 | 43 | 61.0 | 69.8 |
| 14 | 10.0 | 131.5 | 29 | 1.3 | 11.2 | 44 | 791.4 | 612.9 |
| 15 | 14.6 | 191.1 | 30 | 1.9 | 13.2 | 45 | 162.7 | 158.8 |
| | | | | | | 46 | 973.6 | 1234.3 |

### INDUSTRIAL APPLICABILITY

It has been revealed that the inventive compounds have OX receptor antagonist activity. Accordingly, the inventive compounds or pharmaceutically acceptable salts thereof can be used as therapeutic or prophylactic agents for diseases that are regulated by OX receptor antagonist activity, for example, sleep disorder, depression, anxiety disorder, panic disorder, schizophrenia, drug dependence, Alzheimer's disease, Parkinson's disease, Huntington's disease, eating disorder, pain, digestive system disease, epilepsy, inflammation, immune related disease, endocrine related disease, and hypertension.

## Claims

1. A heteroaromatic ring derivative represented by formula (IA): 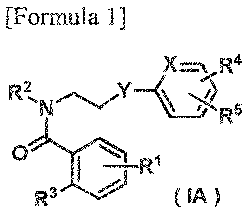 wherein,
X represents a nitrogen atom or formula CH;
Y represents any of the formulas in the following group (II): 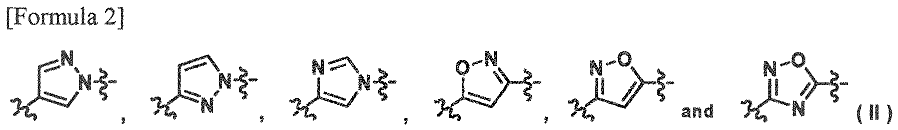
R¹ represents a hydrogen atom, a halogen atom, or a C₁₋₆ alkyl group;
R² represents a hydrogen atom, a C₁₋₆ alkyl group (where, the C₁₋₆ alkyl group may be substituted with one to three substituents selected from Substituent Group 1), a C₃₋₆ cycloalkyl group, or a 4- to 6-membered cyclic ether group, wherein
the Substituent Group 1 is a group consisting of a halogen atom, a C₃₋₆ cycloalkyl group, and a C₁₋₆ alkoxy group;
R³ represents a triazolyl group, a pyridyl group, or a pyrimidinyl group, wherein
the triazolyl group, the pyridyl group, and the pyrimidinyl group are each optionally substituted with one to three halogen atoms; and
R⁴ and R⁵ may be the same or different and each represent a hydrogen atom, a halogen atom, or a C₁₋₆ alkyl group (where, the C₁₋₆ alkyl group is optionally substituted with one to three halogen atoms), or a pharmaceutically acceptable salt thereof.

2. The heteroaromatic ring derivative or a pharmaceutically acceptable salt thereof according to claim 1, wherein
R⁴ represents a halogen atom; and
R⁵ represents a hydrogen atom or a halogen atom.

3. A heteroaromatic ring derivative represented by formula (I): 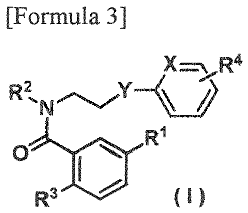 wherein,
X represents a nitrogen atom or formula CH;
Y represents any of the formulas in the following group (II): 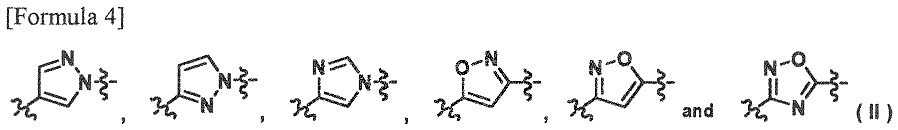
R¹ represents a hydrogen atom, a halogen atom, or a C₁₋₆ alkyl group;
R² represents a hydrogen atom, a C₁₋₆ alkyl group (where, the C₁₋₆ alkyl group is optionally substituted with one to three substituents selected from Substituent Group 1), a C₃₋₆ cycloalkyl group, or a 4- to 6-membered cyclic ether group, wherein
the Substituent Group 1 is a group consisting of a halogen atom, a C₃₋₆ cycloalkyl group, and a C₁₋₆ alkoxy group;
R³ represents a triazolyl group, a pyridyl group, or a pyrimidinyl group, wherein
the triazolyl group, the pyridyl group, and the pyrimidinyl group are each optionally substituted with one to three halogen atoms; and
R⁴ represents a hydrogen atom, a halogen atom, or a C₁₋₆ alkyl group, or a pharmaceutically acceptable salt thereof.

4. The heteroaromatic ring derivative or a pharmaceutically acceptable salt thereof according to any one of claims 1 to 3, wherein
X represents a nitrogen atom;
R² represents a C₁₋₆ alkyl group;
R³ represents a triazolyl group or a pyrimidinyl group; and
R⁴ represents a halogen atom.

5. The heteroaromatic ring derivative or a pharmaceutically acceptable salt thereof according to any one of claims 1 to 4, wherein
R² represents a methyl group or an ethyl group.

6. A pharmaceutical composition comprising a heteroaromatic ring derivative or a pharmaceutically acceptable salt thereof according to any one of claims 1 to 5 as an active ingredient.

7. A therapeutic or prophylactic agent for sleep disorder, depression, anxiety disorder, panic disorder, schizophrenia, drug dependence, Alzheimer's disease, Parkinson's disease, Huntington's disease, eating disorder, pain, digestive system disease, epilepsy, inflammation, immune related disease, endocrine related disease, or hypertension, wherein the agent comprises a heteroaromatic ring derivative or a pharmaceutically acceptable salt thereof according to any one of claims 1 to 5 as an active ingredient.
